# EUROPEAN PATENT APPLICATION

(11) **EP 4 821 368 A1**
(43) Date of publication of application: **14.10.2026**
(21) Application number: 25169254.7
(22) Date of filing: 08.04.2025
(51) Int. Cl.: A24B 13/00, A24B 15/16, A24B 15/30, A61K 31/465

(54) **SMOKELESS ARTICLE, METHOD FOR MAKING A SMOKELESS ARTICLE, AND KIT THEREOF**

(71) Applicant: Imperial Tobacco Limited, Bristol BS3 2LL (GB)
(72) Inventor: HALL, Steven Scott, Bristol, BS3 2LL (GB); LEWERENZ, Nathalie, 22763 Hamburg (DE); FEDORUK, Kateryna, 22763 Hamburg (DE)
(74) Representative: Elkington and Fife LLP

(57) **Abstract**

The present invention relates to a smokeless article for oral consumption comprising a pouch enclosing a content, the content comprising a nicotinic compound, at least one fibrous material, a first flavourant comprising eucalyptus flavourant, a second flavourant comprising a non-eucalyptus flavour component, and a cooling agent distinct from menthol and eucalyptol. A method of manufacturing the articles, and kits comprising the articles, are also described.

## Description

### FIELD

The present disclosure relates to a smokeless article. In particular, the disclosure relates to a smokeless article for oral consumption comprising a pouch enclosing a content, the content comprising a nicotinic compound for oral delivery. This disclosure also relates to methods of manufacturing the smokeless article.

### BACKGROUND

Smokeless articles are a suitable alternative to conventional cigarettes because they do not require heating for substance delivery to the user. Instead, smokeless articles rely on saliva to extract soluble substances, typically nicotine and/or flavours, from the content contained within the smokeless article.

Smokeless articles are placed in the mouth where saliva extracts the soluble element from the content contained within. Typically, the smokeless article is placed in the oral cavity, sublingually or in the oral vestibule (between the teeth and lips/cheeks). The user may assist extraction by oral manipulation, such as by chewing and/or sucking or pressing on the outside of the mouth to squeeze the pouch.

The above-described extraction and delivery process continues until the soluble element is depleted from the smokeless article. The smokeless article is then removed from the mouth and disposed of.

There is a need for improved design of smokeless articles to enhance the user experience and improve the function of its constituent components.

The present disclosure has been devised in the light of the above considerations.

### SUMMARY

At its most general, the present disclosure relates to a smokeless article e.g. an oral nicotine delivery (OND) article, for oral consumption.

**In a first aspect,** the present disclosure provides a smokeless article for oral consumption comprising a pouch enclosing a content. The content is an oral nicotine delivery (OND) formulation. The content comprises a nicotinic compound, at least one fibrous material, a first flavourant, a second flavourant, and a cooling agent. The content may further comprise water. The first flavourant comprises eucalyptus flavourant. The second flavourant comprises a non-eucalyptus flavour component. The cooling agent is distinct from both the eucalyptol and menthol (i.e. it does not comprise eucalyptol or menthol). The first flavourant is present in an amount of between 0.1 and 20 wt% based on the total weight of the composition. The second flavourant is present in an amount of between 0.1 and 20 wt% based on the total weight of the composition. The cooling agent is present in an amount of between 0.01 and 2 wt%.

In some examples, the second flavourant is menthol or comprises a flavour component providing a flavour selected from the group consisting of rosemary, peppermint, frankincense, mint, spearmint, wintergreen, tea tree oil, citrus, oregano, bitter orange, grapefruit, sweet orange, green tea, violet, frankincense, anise, apple, apricot, banana, berries, guava, grape, melon, papaya, peach, pear, peas, clove, ginger, mustard, nuts, honey, coconut, passion fruit, olive, avocado, plum, rose apple, starfruit, sesame seed, tamarind, fig, cardamom, coriander seed and leaf, quince, lychee, dill, lovage, kiwi, loquat, sage, Chinese quince, chilli, nutmeg, cinnamon, lavender, basil, cocoa, vanilla, spice, star anise, fennel, and tobacco. In some examples, the second flavourant is menthol or comprises a flavour component providing a flavour selected from the group consisting of rosemary, peppermint, frankincense, spearmint, wintergreen, tea tree oil, citrus, and oregano. In some examples, the second flavourant is menthol or comprises a flavour component providing a flavour selected from peppermint, spearmint, and wintergreen. In some examples, the second flavourant is menthol.

In some examples, the cooling agent comprises or consists of one or more selected from: WS-3, WS-5, WS-23, WS-12, icilin, camphor, borneol, isoborneol, thymol, and carvone. In some examples, the cooling agent is synthetic. In some examples, the cooling agent has a stronger cooling effect than eucalyptus and/or menthol. In some examples, the cooling agent comprises or consists of WS-5. In this way, it is possible to provide a product with a stronger cooling effect than is ordinarily possible for products comprising eucalyptus and/or menthol as the only cooling components. Therefore, an enhanced cooling and soothing experience can be provided to the user, providing the feeling of mouth freshness.

In some examples, the first flavourant comprises or consists of eucalyptus oil. In some examples, the first flavourant comprises 1,8-cineole (i.e., eucalyptol). In some examples, the first flavourant comprises 70 wt% or more of 1,8-cineole, based on the total weight of the flavourant. In some examples, the first flavourant comprises a synthetic or artificial eucalyptus flavourant.

It has been found that, when a pouch content comprises a combination of a first flavourant comprising eucalyptus flavourant and a second flavourant distinct from eucalyptus, such as menthol or a flavourant providing a flavour selected from the group consisting of rosemary, peppermint, frankincense, mint, spearmint, wintergreen, tea tree oil, citrus, and oregano, the smokeless article can provide a unique flavour combination and organoleptic properties for a favourable and improved user experience. For example, such a combination of flavourants may result in a particularly "fresh" and "cooling" product. Additionally or alternatively, such a combination of flavourants may result in a particularly soothing product and/or a particularly favourable sweetness profile during use (i.e., a temporal soothing profile or sweetness profile). These properties are particularly prominent for the combination of eucalyptus flavourant and menthol or a flavourant providing a mint, spearmint, or peppermint flavour. It has been found that such properties are particularly prevalent, or even enhanced, when an additional cooling agent is also included, in particular a cooling agent other than eucalyptus or menthol. That is, an oral product having a combination of a eucalyptus flavourant, a second flavourant (such as menthol), and a cooling agent other than eucalyptus or menthol may provide a particularly "fresh" and "cooling" user experience, with a unique time-dependent flavour and/or aroma distribution as well as providing soothing to the user's mouth.

Eucalyptus, when part of a content for oral consumption, can provide a refreshing sensation due to its cooling and soothing properties and desirable aromatic profile. The pleasant flavour and aroma of eucalyptus can stimulate a mental association with the cooling and soothing nature associated with eucalyptus. Such soothing, cooling, and aromatic sensations, and in particular the soothing sensations, are thought to be at least partly due to a user mental association between eucalyptus and soothing properties. Thus, when in use a user tastes or smells a content or otherwise understands that a content comprises eucalyptus, a mental association between the product and eucalyptus may induce to a perceivable soothing and/or cooling sensation during delivery of the smokeless article, thereby providing a favourable user experience.

Additionally, natural eucalyptus (defined below) comprises eucalyptus compounds which, alone or in combination, are also thought to further contribute towards the above-mentioned soothing, cooling, and aromatic sensations. One such eucalyptus compound is 1,8-cineole, which forms the major component of eucalyptus oil. Without wishing to be bound by theory, it is thought that eucalyptus compounds contribute towards such sensations due to chemical interactions in the user's mouth (e.g., proximal to a user's gums). By virtue of chemical interactions with a user, eucalyptus compounds may contribute towards cooling and soothing sensations in a way distinct from, and possibly to a greater extent than, a mental association between eucalyptus and a smokeless article (e.g., in terms of its flavour, aroma, or otherwise).

When eucalyptus oil or cineole is included in the smokeless article, the eucalyptus component can provide a "cooling" or "tingling" sensation due to the stimulation of sensory nerves in the mouth by compounds present in eucalyptus oil, such as 1,8-cineole. This results in a pleasant user experience. The oxygenated monoterpene eucalyptol (also known as1,8-cineole) is the primary element shared by all Eucalyptus species. Eucalyptol has a fresh camphor-like odour and a spicy, cooling taste. Due to its cooling effect, it may also help soothe the throat. Additionally, the cooling effect may provide a soothing sensation to users who experience sensitivity, irritation, or burning when using nicotine products.

When a second flavourant is also included in the article for oral consumption, all the above benefits of eucalyptus can be achieved and even enhanced through the addition of another flavour, aroma or sensory profile. For example, the perceived freshness can be enhanced by the addition of another flavourant which induces freshness (e.g., one of rosemary, peppermint, frankincense, menthol, spearmint, wintergreen, tea tree oil, citrus, and oregano). The user is able to access a broad spectrum of taste and aroma when the product comprises a first, eucalyptus flavourant, a second non-eucalyptus flavour component, and an additional cooling agent. The second flavourant may have overlapping or similar properties to eucalyptus to enhance the existing eucalyptus experience. Alternatively, the second flavourant may have distinct or contrasting properties to eucalyptus, to provide a unique and broader sensory experience, for example by providing a heating or warming sensation. By including a second (and optionally third) flavourant and a further cooling agent, the user experience can be greatly improved and tailored to provide a unique experience, whilst still benefiting from the effects of eucalyptus (e.g., the cooling and/or soothing effects).

Additionally, the second flavourant may be realised by the user on a different time-frame to the first flavourant comprising eucalyptus. For example, the user may initially taste eucalyptus followed by the second flavourant, or vice-versa. This can provide a unique time-dependent flavour and/or aroma distribution during an active lifetime of the smokeless article. The unique time-dependent flavour and/or aroma distribution(s) may, at least in part, relate to a temporal sweetness profile. Such combinations may additionally exhibit a unique time-dependent physiological experience concurrent with the flavour distribution, for example a favourable tingling or soothing sensation. The unique flavour distributions and physiological combinations exhibited by combinations of the present invention may not be exhibited by known flavour components, alone or in combination.

The second flavourant may be selected for its cooling, tingling, warming, or numbing sensation, for its pleasant taste, such as sweet, spicy or sour, and/or for its pleasant aroma.

In some examples, the second flavourant comprises a single botanical. In some examples, the second flavourant comprises a botanical of only one plant species or hybrid. In this way, the specific flavour combination and the interplay between eucalyptus, the second flavourant, and the cooling agent can be precisely controlled and intimately paired.

In some examples, the second flavourant comprises or consists of a mint flavourant. In some examples, the second flavourant comprises or consists of one or more of a mint botanical, menthol (e.g. L-menthol), or a menthol derivative. In some examples, the second flavourant comprises peppermint or spearmint, such as a flavourant providing a peppermint or spearmint flavour. In some examples, the second flavourant comprises or consists of menthol or a menthol derivative such as menthyl lactate or menthoxypropanediol. In this way, the cooling sensation in the user's mouth may be enhanced, for example by dual action on the TRPM8 protein. Additionally, an oral product having a mint or menthol flavour component as well as a eucalyptus flavourant and an additional cooling agent can result in a substantially greater cooling and soothing sensation, thus amplifying the user's pleasurable experience.

In some examples, the second flavourant comprises or consists of menthol and the cooling agent comprises or consists of WS-5.

In some examples, the smokeless article additionally comprises a third flavourant. The third flavourant may be any flavourant as described for the second flavourant herein. When included, the third flavourant is different to the first flavourant and the second flavourant. In this way, and even broader taste and sensory profile can be achieved. The third flavourant may be selected to enhance the freshness and cooling sensation. The third flavourant may be selected for its cooling, tingling, warming, or numbing sensation, for its pleasant taste, such as sweet, spicy or sour, and/or for its pleasant aroma. In some examples, the third flavourant comprises a single botanical. In some examples, the third flavourant comprises a botanical of only one plant species or hybrid. In this way, the specific flavour combination and the interplay between eucalyptus, the second flavourant, the cooling agent, and the third component can be precisely controlled and intimately paired.

The second and third flavourants described herein may also each contribute towards and enhance a perceived cooling, soothing, and/or aromatic sensation by association, or due to a second flavourant's unique blend of components which may chemically interact in a user's mouth during delivery. When an additional flavourant is present in combination with eucalyptus flavour as part of a content for oral delivery, the content may therefore exhibit particularly soothing and aromatic properties, such as scent and flavour, for a favourable user experience. That is, all the above effects associated with eucalyptus flavour may be achieved and even enhanced when a second flavourant is present.

To further elaborate, it has been found that such flavourant combinations with eucalyptus flavour may exhibit unique and particularly pleasant physiological experiences, such as soothing and cooling sensations during an active lifetime of the smokeless article (during use), which may be time-dependent. The unique time-dependent soothing and cooling sensations may be concurrent with a particularly unique and pleasant time-dependent (temporal) mouthfeel, such as a particularly smooth mouthfeel that is maintained during the active lifetime of the smokeless article. The unique flavour and aroma distributions, and physiological sensations exhibited by combinations of the eucalyptus flavour and additional flavourants may not be exhibited by known flavourants, alone or in combination. In other words, by providing a content comprising a eucalyptus flavourant and one or more additional flavourants as defined herein, a user experience can be greatly improved and tailored to provide a unique experience, whilst still benefiting from the effects of eucalyptus flavour (e.g., soothing effects, induced by mental association and/or chemical interaction with a user during use).

For example, when a eucalyptus flavourant is combined with a second flavourant and/or a cooling agent defined herein, a user may for example experience an initial high intensity of soothing and cooling (or 'freshness') and a sustained soothing sensation, or favourable variations in soothing sensations during delivery of the smokeless article which are otherwise inaccessible. Such sensations may be concurrent with a sustained sweetness profile, which may increase or decrease in intensity during use, as component compounds responsible for soothing are released from the content. Alternatively, for some combinations, a user may experience a low-intensity initial soothing sensation that increases during use, possibly concurrently with an initial high intensity of sweetness and a sustained sweetness experience.

In some examples, such as those containing a third flavourant, the amount of each flavourant is independently between 0.1 and 10 wt%, based on the total weight of the content. In some examples, the amount of each flavourant is independently between 0.1 and 20 wt%, and the total amount of flavourant (i.e. the sum of the amounts of each flavourant) is between 0.1 and 20 wt%, optionally between 0.1 and 10 wt%. In some examples, the amount of each flavourant is independently between 0.1 and 10 wt%, and the total amount of flavourant (i.e. the sum of the amounts of each flavourant) is between 0.1 and 20 wt%, optionally between 0.1 and 10 wt%.

In some examples, the weight ratio of the second flavourant to the first flavourant (i.e. [amount by weight of second flavourant in content] / [amount by weight of first flavourant in content]) is between 0.01 and 100. In some examples, the ratio is between 0.1 and 10. In this way, the desired properties of each flavourant can simultaneously be achieved.

Generally, 'eucalyptus', when referring to a plant, refers to any plant of the *Eucalyptus* genus. In the context of flavourants, eucalyptus may refer to plant products of (i.e., botanicals of) a *Eucalyptus* plant, wherein said botanicals or products comprise eucalyptus compounds, or it may refer to a constituent, derivative, isolate, or mimetic of a *Eucalyptus* plant or eucalyptus compound.

Correspondingly, in some cases, the flavour component of the second flavourant may refer to a botanical or product of a plant (i.e. a flavour component providing a flavour of said plant). For example, a rosemary flavour component may be a rosemary botanical or plant product (e.g., a plant product or botanical of the rosemary plant, *Salvia Rosmarinus*).

As used herein, "natural eucalyptus" is intended to mean any constituent, extract, or a mixture thereof, obtained from, or which can otherwise be found in, plants of the *Eucalyptus* genus. A "eucalyptus compound" refers to a compound which is naturally present in any plant of the *Eucalyptus* genus. A eucalyptus compound may be characteristic of a *Eucalyptus* plant, such as eucalyptol (aka 1,8-cineole).

As used herein, "synthetic eucalyptus" is intended to mean a mimetic, derivative, or analogue of a constituent, derivative, extract, or a mixture thereof, which can be found in plants of the *Eucalyptus* genus, or a compound which provides similar properties (e.g. flavour and/or aroma) to a eucalyptus compound but is not found in *Eucalyptus* plants. For example, synthetic eucalyptus encompasses a synthetic analogue of a eucalyptus compound, as well as a compound having predominantly similar properties as a eucalyptus compound such as aroma and/or taste, but otherwise not found in *Eucalyptus* (e.g., a compound having a eucalyptus flavour - see the definition of "artificial eucalyptus" below). For the avoidance of doubt, synthetic eucalyptus may include a compound which is made synthetically but is otherwise available naturally. Additionally, synthetic eucalyptus may use natural precursors or starting materials.

Synthetic eucalyptus also encompasses "artificial eucalyptus" compounds which are compounds not naturally found in *Eucalyptus* plants. Artificial eucalyptus compounds share one or more other similar properties to a eucalyptus compound, such as flavour and/or aroma, but they may be structurally related or structurally unrelated to natural eucalyptus compounds. For example, an artificial eucalyptus flavourant may be a flavourant or compound which provides the flavour and/or aroma of eucalyptus but is not a natural eucalyptus compound. For the avoidance of doubt, a (chemically modified) derivative of a natural eucalyptus compound may be considered both a synthetic and artificial eucalyptus compound.

"Eucalyptus flavourant" may refer to natural eucalyptus and/or synthetic eucalyptus (including artificial eucalyptus).

As used herein, 'botanical' or 'plant product' may refer to any material derived from a plant including, but not limited to, extracts, leaves, bark, fibres, stems, roots, seeds, flowers, fruits, pollen, husk, shells or the like. The material may be in the form of liquid, gas, solid, gel, powder, dust, crushed particles, granules, pellets, shreds, strips, sheets, or the like. In various embodiments of the present disclosure the material is in the form of liquid, solid, gel, powder, crushed particles, granules, pellets, shreds, strips, sheets or the like. In particular, 'botanical' or 'plant product' may refer to, an extract, oil (such as an essential oil), butter, powder, gel, and concentrate. By way of example, eucalyptus flavourant(s) may refer to *Eucalyptus* botanicals and/or plant products, including eucalyptus extract, eucalyptus oil, eucalyptol, and/or eucalyptus concentrate.

As used herein, an "extract" is a product that is obtained through an extraction and/or separation process where plant material is used as raw materials. Generally, the original components of the plant material are not changed. The extract may be purified. In some examples, the extract is processed to remove unwanted components. In other examples, the extract is not processed to remove any specific components. The extract may be an essential oil.

Essential oils are oils extracted from plant material. They typically have an aroma and/or flavour which is characteristic of the plant from which they are obtained (i.e., they provide a flavour of the plant from which they are obtained). They are known to be used as flavourants. The extraction method of essential oil can be divided into two categories, conventional and modern methods. Conventional methods include hydro-distillation, steam distillation, and extraction using solvents. Modern methods include supercritical fluid extraction, microwave-assisted hydro-distillation, and ultrasound-assisted extraction. Essential oils from many plants are commercially available.

As used herein, a "constituent" of a plant is a compound or component found naturally in said plant., for example a compound or component found in an extract. Examples of constituents include volatile organic compounds, proteins, lipids, solvents, oils, esters, alcohols, and the like.

As used herein, a "derivative" is compound which is modified after isolation. For example, a derivative of a plant product may be a chemically modified constituent of the plant product. Modification may be by a chemical reaction, for example a condensation reaction, a substitution reaction, or a hydrolysis reaction.

Certain constituents, derivatives, or extracts may be selected based on their specific properties, such as taste, aroma, inertness, cooling effect, soothing effect, etc.

As used herein, the term "oral consumption" is intended to refer to any oral administration route achieved by placing the smokeless article into the oral cavity. This includes, but is not limited to, buccal, sub-lingual, periodontal, gingival and ingestion.

As used herein, the term "flavourant" denotes a compound having a desirable taste, aroma or both.

As used herein, "freshness" refers to the quality of something being perceived as fresh. This may be associated with a "cool" and/or "clean" taste or sensation. For example, it may be associated with a cooling sensation within a user's mouth. It may also be associated with a decongestant.

As used herein, "cooling" refers to a sensation of cold in a user's mouth. This may be caused by activation of proteins associated with perception of cold, such as TRPM8 proteins. It may be associated with a "clean" and/or "fresh" taste or sensation. It may also be associated with a decongestant.

**The smokeless article** may be an oral nicotine delivery (OND) article when the active agent within the content comprises a nicotinic compound.

In some examples, the active agent comprises or consists of an active compound. In some examples the active agent comprises or consists of a nicotinic compound.

The smokeless article comprises a pouch enclosing a content, wherein the content (including e.g. a nicotinic compound and non-tobacco plant material fibres) is completely enclosed by the pouch. The pouch is sealed to ensure that the contents of the pouch does not scatter inside the mouth.

The smokeless article may have a mass of from about 0.1 g to about 5.0 g. For example, the smokeless article may have a mass of from about 0.2 g to about 3.0 g, from about 0.25 g to about 2.0 g, from about 0.3 g to about 1.5 g, from about 0.4 g to about 1.2 g, from about 0.5 g to about 1.1 g, from about 0.6 g to about 1.0 g, or from about 0.6 g to about 0.9 g. In some cases, the mass is about 0.6 g, about 0.7 g, about 0.8 g, or about 0.9 g.

The smokeless article may have a length of from about 25 mm to about 40 mm. For example, the length may be from about 28 mm to about 40 mm, from about 29 mm to about 39 mm, or from about 30 mm to about 38 mm. In some cases, the length is from about 32 mm to about 38 mm. In some cases, the length may be about 30 mm, about 32 mm, about 34 mm, about 36 mm, or about 38 mm.

The smokeless article may have a width of from about 8 mm to about 20 mm. For example, the width may be from about 9 mm to about 19 mm, from about 10 mm to about 18 mm, or from about 12 mm to about 16 mm. In some cases, the width is from about 10 mm to about 18 mm. In some cases, the width may be about 10 mm, about 12 mm, about 14 mm, about 16 mm, or about 18 mm.

The smokeless article may have a depth of from about 1 mm to about 8 mm. For example, the width may be from about 2 mm to about 7 mm, or from about 2 mm to about 6 mm. In some cases, the depth is about 3 mm, about 4 mm, about 5 mm, or about 6 mm.

The smokeless article may have an active lifetime of about 10 minutes to about 60 minutes, such as about 15 minutes to about 55 minutes, about 20 minutes to about 50 minutes, about 25 minutes to 50 minutes or about 30 minutes to about 45 minutes, after being placed in the mouth. As used herein, the term "active lifetime" is intended to refer to the amount of time after being placed in the mouth that the smokeless article provides the user with a perceptible taste and/or physiological experience. For example, for an article containing an active ingredient such as nicotine or other pharmacologically active ingredient the active lifetime may be defined as the in use period of time in which 90%wt of the available pharmacologically active is released. In other words, the active lifetime may be the duration of time from insertion into the oral cavity for 90%wt of the total amount of nicotine pharmacologically active ingredient that is capable of being released during normal use to dissolve into the user's saliva and /or enter the user's bloodstream. It will therefore be appreciated that the active lifetime of a product may vary from user to user and for a user based on oral conditions, in particular extent of salivation. Nonetheless, the skilled person is able to mimic oral conditions to determine the active lifetime in one instance, which can be used as a comparison or analysis point.

**The pouch** may be formed from one or more materials. The pouch material may be formed from fiber, paper, cloth and fabric. The pouch material may be formed from one or more polymeric materials. The polymeric material may be selected from one or more of viscose, polyester, hydroxypropyl cellulose (HPC), hydroxypropyl methylcellulose (HPMC), polyvinyl alcohol (PVOH), polyvinylpyrrolidone (PVP), polyethylene oxide (PEO) hydroxyethyl cellulose (HEC), polyethylene glycol (PEG), pullulan, sodium alginate, xanthan gum, tragancanth gum, guar gum, acacia gum, arabic gum, polyacrylic acid, maltodextrin, methylmethacrylate copolymer, carboxyvinyl copolymers, starch and gelatin. The pouch material may comprise a binder, for example in addition to the polymeric material. Suitable binders include, for example, acrylic binders and synthetic latex.

In some examples, the pouch material comprises viscose (e.g. Rayon). Viscose may be made from natural sources of regenerated cellulose, such as wood and related agricultural products. In some examples, the pouch material comprises viscose and an additional polymer and/or binder. For example, the pouch material may comprise viscose and polyester, viscose and acrylic binder, or viscose and synthetic latex. In some examples, the pouch material comprises viscose and synthetic latex.

The pouch is typically completely insoluble in saliva. Suitable insoluble pouch materials include, but are not limited to, fiber, paper, water-insoluble polymers, cloth and fabric. Suitable soluble pouch materials include, but are not limited to, water-soluble polymers such as polyethylene oxide (PEO), hydroxypropyl cellulose (HPC) and hydroxypropyl methylcellulose (HPMC).

The pouch may be formed by, for example, folding a single sheet on itself or bringing two or more sheets together and sealing the edges. The edges may initially be partially sealed to provide an open pouch in which the content (e.g. a nicotinic compound and non-tobacco plant material fibres) may be placed before completely sealing the pouch closed. The sheets may be the same thickness or different thicknesses.

The pouch is porous. In some examples, at least 50% of the pores have a diameter of 50 µm to 200 µm, such as 100 µm to 175 µm or 125 µm or 150 µm. In some examples, at least 50% of the pores have a diameter of at least 100 µm. For example, in some examples at least 55%, 60%, 65%, 70%, 75%, 80%, 85%, 90%, 95% or 100% of the pores have such diameters.

The pouch may be coloured or include markings, such as brand logos and text, to improve user perception. The pouch may be partially or completely coloured by a colourant.

In some examples, the active agent comprises or consists of a nicotinic compound.

The nicotinic compound may be selected from nicotine, nicotine salt(s), nicotine complex(es); and nicotine solvate(s).

In some examples, the smokeless article is tobacco free. In this way, the user may experience a similar or enhanced recreational/pharmaceutical effect as compared to conventional tobacco-containing products without experiencing undesirable components inherent to tobacco (e.g. tobacco flavour).

In some examples, the content comprises a nicotinic compound (e.g. nicotine) in an amount of from 0.3 wt% to 2 wt%, for example from 0.4 wt% to 2 wt%, from 0.5 wt% to 2 wt%, from 0.5 wt% to 1.9 wt%, from 0.8 wt% to 1.8 wt%, from 1 wt% to 1.6 wt%, from 1.2 wt% to 1.6 wt%, or from 1.3 wt% to 1.5 wt%, based on the total weight of the content.

In some examples, the total nicotinic compound (e.g. nicotine) content per pouch is from 4 to 15 mg. In some examples, the nicotinic compound content per pouch is about 7-10 mg, for example the nicotinic compound content per pouch is about 8 mg.

The content of the smokeless article may comprise water. In some examples, the amount of water in the content is from about 1 wt% to about 65 wt%.

In some examples, the amount of water in the content is from about 25 wt% to about 65 wt%. For example, the amount of water may be from about 25 wt% to about 60 wt%, from about 25 wt% to about 55wt%, from about 28 wt% to about 51 wt%, from about 30 wt% to about 50 wt%, or from about 33 wt% to about 48 wt%. In some cases, the amount of water is from about 33 wt% to about 41 wt%. In some cases, the amount of water is from about 41 wt% to about 49 wt%. In some cases, the amount of water is about 30 wt%, about 33 wt%, about 36 wt%, about 38 wt%, about 40 wt%, about 42 wt%, about 44 wt%, about 46 wt%, about 48 wt%, or about 50 wt%.

In some examples, the amount of water in the content is from about 1 wt% to about 25 wt%. For example, the amount of water may be from about 1 wt% to about 20 wt%, from about 1 wt% to about 15 wt%, from about 2 wt% to about 13 wt%, from about 3 wt% to about 12 wt%, from about 4 wt% to about 11 wt%, or from about 5 wt% to about 10 wt%. In some cases, the amount of water is about 1 wt%, about 2 wt%, about 3 wt%, about 4 wt%, about 5 wt%, about 6 wt%, about 7 wt%, about 8 wt%, about 9 wt%, or about 10 wt%.

The content may comprise one or more additional substance. Each additional substance may individually be a biologically/pharmacologically active compound, humectants, flavourants, fillers, preservatives, aqueous/non-aqueous solvents and binders. Each additional substance may be provided for more than one purpose.

The contents of the pouch (i.e. the ingredients, material and/or substances enclosed within the pouch) preferably occupies substantially all of the internal volume of the pouch. The contents may occupy 80%, 85%, 90%, 95% or 100% of the internal volume of the pouch. The contents may comprise a solid material to provide physical integrity, such as an organic material (e.g. plant material) or an inorganic material. Such solid materials may naturally or inherently contain one or more biologically/pharmacologically active compounds and/or additives.

**A first flavourant** comprising eucalyptus flavourant is included in the content. A eucalyptus flavourant is a flavourant which provides a flavour and/or aroma and/or organoleptic sensation associated with eucalyptus. It may be natural or synthetic.

In some examples, the eucalyptus flavourant comprises or consists of a natural eucalyptus flavourant. In some examples, the eucalyptus flavourant consists of a natural eucalyptus flavourant. In some examples, the natural eucalyptus flavourant is an extract from *Eucalyptus* plant material. In some examples, the eucalyptus flavourant is a constituent of *Eucalyptus* plant material, such as one or more compounds found in *Eucalyptus.* In some examples, the eucalyptus flavourant is a derivative of *Eucalyptus* plant material, such as a chemical derivative of one or more compounds found in *Eucalyptus* (e.g. a chemical derivative of one or more *Eucalyptus* constituents). In some examples, the eucalyptus flavourant is eucalyptus oil.

The *Eucalyptus* genus is a member of the *Eucalyptae* tribe, and a member of the *Myrtaceae* family. There are over 700 species within the *Eucalyptus* genus, any of which, or any hybrids thereof, may be used as, or as a source of, the eucalyptus flavourant of the first flavourant. Examples of common *Eucalyptus* species which may be used in the first flavourant include, but are not limited to, *Eucalyptus globulus, Eucalyptus gunnii, Eucalyptus cneorifolia, Eucalyptus dives, Eucalyptus dumosa, Eucalyptus goniocalyx, Eucalyptus horistes, Eucalyptus kochii, Eucalyptus leucoxylon, Eucalyptus oleosa, Eucalyptus radiata, Eucalyptus sideroxylon, Eucalyptus smithii, Eucalyptus tereticornis, Eucalyptus viridis, Eucalyptus pauciflora, Eucalyptus polyanthemos, Eucalyptus camaldulensis, Eucalyptus pulchella, Eucalyptus conferruminata, Eucalyptus polybractea, Eucalyptus dalrympleana, Eucalyptus deglupta, Eucalyptus perriniana, Eucalyptus neglecta, Eucalyptus rubida, Eucalyptus astringens,* and *Eucalyptus nicholii.* In some examples, the eucalyptus flavourant is, or is obtained from, *Eucalyptus globulus* and/or *Eucalyptus gunnii.*

The eucalyptus flavourant included in the first flavourant may be a eucalyptus constituent, derivative, or extract, or a mixture thereof. For example, the eucalyptus flavourant may be a constituent, derivative, or extract obtained from eucalyptus plant matter.

Eucalyptus leaves contain, *inter alia,* flavonoids (plant-based antioxidants), volatile oils, and tannins. Eucalyptus oil is commonly used as an herbal medicine due to its composition of, e.g., esters, aldehydes, ketones, monoterpenes, and sesquiterpenes. The oxygenated and unoxygenated monoterpenes, as well as the oxygenated sesquiterpenes, make up the majority of the essential oil.

The oxygenated monoterpene eucalyptol (also known as 1,8-cineole) is the primary element shared by all Eucalyptus species.

The eucalyptus flavourant may be provided as a raw material or a processed material. A raw material may be one which has not been chemically processed, though it may have been mechanically processed. A processed material may be one which has been chemically processed in some way.

In the case of a eucalyptus constituent, derivative, or extract, the eucalyptus may be from natural sources (e.g., isolated from plant matter) or it may be from synthetic sources (e.g., synthetic eucalyptol) - in either case, such eucalyptus flavourants are commonly available from commercial sources. In the case of the eucalyptus flavourant being from natural sources, the eucalyptus component may be obtained from any material derived from a eucalyptus plant, including but not limited to, extracts, leaves, bark, fibres, stems, roots, seeds, flowers, fruits, pollen, husk, shells or the like. The plant material may be in the form of liquid, gas, solid, gel, powder, dust, crushed particles, granules, pellets, shreds, strips, sheets, or the like.

Additionally or alternatively, eucalyptus flavourants may comprise eucalyptus compounds that are obtained synthetically. Such synthetic eucalyptus compounds may be known to be present in the *Eucalyptus* plant (and in turn in natural eucalyptus flavourants), but may be produced using synthetic methods. The eucalyptus flavourants may comprise such 'synthetic' eucalyptus compounds, as an alternative to or in addition to 'natural' eucalyptus compounds being obtained from the *Eucalyptus* plant using isolation and/or purification methods. Some such eucalyptus compounds may be commercially available. For example, synthetic eucalyptol (i.e., 1,8-cineole) is commercially available. In some examples, the first component comprises or consists of a synthetic eucalyptus flavourant. In some examples, the first component consists of a synthetic eucalyptus flavourant.

Additionally or alternatively, eucalyptus flavourants may comprise a mimetic, derivatives, or analogues of a constituent, derivative, extract, or a mixture thereof, which can be found in plants of the *Eucalyptus* genus (for example, a synthetic analogue of a eucalyptus compound, or a compound having predominantly similar properties as a eucalyptus compound such as aroma, taste, and/or cooling properties). In some examples, the eucalyptus flavourant may comprise artificial eucalyptus which is not naturally present in *Eucalyptus* plants, but which is structurally similar or which shares similar physical or aromatic properties to a eucalyptus compound. For example, a synthetic eucalyptus flavourant (e.g. artificial eucalyptus) may possess a similar taste and/or aroma profile to *Eucalyptus*, but it may not be naturally present in *Eucalyptus.* Such a synthetic eucalyptus flavourant may or may not be structurally similar to a natural eucalyptus compound.

Eucalyptus flavourants may additionally or alternatively comprise eucalyptus compound derivatives or variants. 'Eucalyptus compound derivative' means a eucalyptus compound which is modified after isolation. Modification may be by a chemical reaction, for example a condensation reaction, a substitution reaction, or a hydrolysis reaction. 'Eucalyptus compound variant' refers to a synthetic variant (analogue) of a known naturally occurring eucalyptus compound.

In some examples, the eucalyptus flavourant is in the form of eucalyptus oil. In some examples, the eucalyptus flavourant consists of eucalyptus oil. In some examples, the first flavourant comprises or consists of eucalyptus oil. In some examples, the first flavourant comprises eucalyptus oil. In some examples, the first flavourant consists of eucalyptus oil.

Eucalyptus oil may be obtained from the leaf of a eucalyptus leaf (i.e. the leaf of a species of the *Eucalyptus* genus). In some examples, the eucalyptus oil is distilled. In some examples, the eucalyptus oil is refined. In some examples, the eucalyptus oil is produced by refining or distilling the raw eucalyptus oil, for example to isolate, remove, or concentrate a specific component of the eucalyptus oil, such as eucalyptol. In some examples, the eucalyptus oil is pharmaceutical grade, defined by the British Pharmacopoeia as having a minimum 1,8-cineole content of 70%. In some examples, the first flavourant comprises 70 wt% or more, 75 wt% or more, 80 wt% or more, 85 wt% or more, 90 wt% or more, or 95 wt% or more of 1,8-cineole, based on the total weight of the first flavourant. In some examples, the first flavourant consists essentially of 1,8-cineole.

In some examples, the eucalyptus flavourant comprises or consists of eucalyptol. Eucalyptol is 1,8-cineole (also referred to herein as "cineole"). Eucalyptol is a constituent of *Eucalyptus* plant material and is found in eucalyptus oil. It may also be considered an extract of *Eucalyptus* plant material, such as from *Eucalyptus* leaves. In some examples, the cineole is natural. In some examples, the cineole is synthetic. Natural cineole may be obtained from *Eucalyptus* plant material, for example by distillation of eucalyptus oil or by any other isolation or purification method known in the art (e.g. chromatography). Synthetic cineole may be produced by synthetic methods known in the art, such as by isomerization of α-terpineol catalyzed by heteropoly acid H₃PW₁₂O₄₀. The chemical structure of 1,8-cineole, characterized by its cyclic ether containing a three-atom ring, makes it highly effective in crossing biological barriers. Cineole has a fresh camphor-like odour and a spicy, cooling taste. Cineole acts as a cooling agent which can provide a cooling and/or soothing sensation. As a result, it can provide a pleasant organoleptic sensation to users, for example to those who experience irritation and/or burning due to nicotine products.

Eucalyptol has a pleasant aroma and taste which can be imparted to a smokeless article to improve the user experience. It has a fresh smell and elicits a cooling sensation in the mouth. It is reported that the cooling sensation may be the result of activation of the TRPM8 protein by cineole, which may be associated with increased user energy and alertness, as well as increased flavour perception. Without wishing to be bound by theory, it has been suggested that cineole may inhibit sensory irritation caused by octanol and menthol in users. This suggests that combining cineole with a second flavourant containing octanol and menthol may provide a favourable user experience and taste profile for users who would otherwise avoid such flavours (Takaishi M *et al.,* "1,8-cineole, a TRPM8 agonist, is a novel natural antagonist of human TRPA1." *Mol Pain.* 2012;**29:**8-86. doi: 10.1186/1744-8069-8-86). Menthol is a component of *Mentha* plants, and is found in common flavourants peppermint and spearmint, and octanol is a component of oils of botanicals used as common flavourants, such as bitter orange, grapefruit, sweet orange, green tea, violet and frankincense. Hence, the combination of cineole and one or more of these flavourants could result in the abovementioned beneficial effect. Similar effects can also be expected when a menthol derivative is used as the second flavourant, such as menthyl lactate or menthoxypropanediol. Additionally, 1-octanol has also been reported to be found in apple, apricot, banana, citrus peel oils and juices, berries, guava, grape, melon, papaya, peach, pear, peas, clove, ginger, mustard, spearmint oil, nuts, honey, coconut, passion fruit, olive, avocado, plum, rose apple, starfruit, sesame seed, tamarind, fig, cardamom, coriander seed and leaf, quince, lychee, dill, lovage, kiwi, loquat, sage, and Chinese quince. Therefore, combining any of these flavours with eucalyptus (e.g. cineole) may provide a more enjoyable user experience.

Since cineole is a major component of eucalyptus oil, the above benefits can be achieved by a range of eucalyptus constituents, derivatives, extracts, or mixtures thereof.

The first flavourant may include any other compound found in *Eucalyptus,* either alone or in combination with one another or in combination with cineole. For example, the first flavourant may comprise cineole and one or more other compounds found in *Eucalyptus* (i.e., eucalyptus compounds). Such other compounds (which may be referred to as constituents of *Eucalyptus*) include, for example, macrocarpal-C, p-cymene, α and β-pinene, limonene, citronellal, citral, eudesmol, terpinen-4-ol, terpineol, α-phellanderene, and 9β-sitosterol. Each of these may be selected for their one or more properties, such as aroma, flavour, or cooling effect. In some examples, the eucalyptus comprises macrocarpal C. Macrocarpal C is found in certain *Eucalyptus* leaves (e.g., *E*. *macrocarpa* and *E*. *camaldulensis*), hence it is a constituent of *Eucalyptus* and may be present in extracts of *Eucalyptus.* Macrocarpal C is not typically found in certain species of *Eucalyptus,* including *E. globulus* (which is a common source of eucalyptus oil), therefore it may be desired to add a supplemental amount of macrocarpal C to impart its desirable properties. Macrocarpal C can be isolated from fresh *Eucalyptus* leaves by ethanolic extraction, followed by drying, followed by *n-*hexane solvent separation, followed by chromatographic purification.

In some examples, the eucalyptus flavourant comprises an extract of *Eucalyptus* to which an additional amount of a *Eucalyptus* constituent has been added. This may be described as a eucalyptus flavourant comprising supplemental eucalyptus compounds. In some examples, the eucalyptus flavourant comprises eucalyptus oil to which an additional amount of a *Eucalyptus* constituent has been added. In some examples, the eucalyptus flavourant may comprise eucalyptus oil with additional 1,8-cineole. In some examples, the eucalyptus flavourant may comprise eucalyptus oil with additional macrocarpal C. In some examples, the eucalyptus flavourant may comprise eucalyptus oil with additional pinene. In some examples, the eucalyptus flavourant may comprise eucalyptus oil with additional limonene.

In some examples, the eucalyptus flavourant comprises natural eucalyptus and synthetic eucalyptus. For example, the eucalyptus flavourant may comprise natural eucalyptus to which an amount of an artificial eucalyptus flavourant is added (such as a synthetic compound which provides or mimics the flavour of eucalyptus but is not naturally found in *Eucalyptus* plants). This may be referred to as flavour-enhanced natural eucalyptus.

In some examples, the first flavourant comprises a eucalyptus flavourant and one or more additional flavour components. In some examples, the first flavourant comprises a eucalyptus flavourant and menthol. That is, the first flavourant, prior to being included in the content comprises a eucalyptus flavourant and menthol, separate to any subsequent or supplemental menthol which may or may not be included as a second or third flavourant. Therefore, in some examples, the first flavourant comprises a eucalyptus flavourant and menthol, and the second flavourant comprises or consists of menthol. In such a case, the menthol of the second flavourant may be referred to as supplemental menthol. In this way, the cooling effect and freshness of a eucalyptus flavourant comprising menthol (such as some commercially available eucalyptus flavourants which are provided already containing menthol) can be enhanced by the addition of supplemental menthol.

In some examples, the first flavourant does not contain liquorice or a flavourant providing the flavour of liquorice. In some examples, the smokeless article does not contain liquorice or a flavourant providing the flavour of liquorice. Some commercially available eucalyptus flavourants are known to contain an amount of liquorice flavourant (such as a liquorice extract). Without wishing to be bound by theory, it is thought that a smokeless article which does not contain liquorice may provide a more desirable cooling and taste experience compared to one containing liquorice. This may be because liquorice possesses a dulling and numbing effect in the user's mouth, which may be due to the presence of glycyrrhizin in liquorice plants.

In some examples, the flavourant is provided in solid form. In some examples, the flavourant is provided in liquid form. In some examples, the flavourant is provided as a solution. For example, the flavourant may be provided as a solution in one or more of water, ethanol, propylene glycol, or vegetable glycerine. In some examples, the flavourant is provided as a gel. In some examples, the flavourant is provided as an oil. In some examples, the flavourant consists of a single compound (e.g. 1,8-cineole). In some examples, the flavourant is adhered to a solid substrate. In some examples, the flavourant is provided in raw form (i.e., it does not comprise any additional additives). In some examples, the flavourant does not comprise a solvent. In some examples, the flavourant does not comprise an added solvent (i.e., a solvent other than those which are inherent to the flavourant, such as those found naturally in a botanical flavourant).

In some examples, the first flavourant consists of eucalyptus flavourant. In some examples, the first flavourant consists only of one or more *Eucalyptus* constituents, derivatives, extracts, or mixtures thereof, such as eucalyptus oil. In other words, in some examples, the first flavourant does not comprise any additional components which are not obtained from or found in *Eucalyptus* plants. In some examples, the first flavourant consists of natural eucalyptus and/or synthetic eucalyptus.

In some examples, the first flavourant consists of eucalyptus flavourant and one or more excipients, diluents, or solvents. Suitable excipients, diluents, and solvent for flavour compositions are known to those skilled in the art. For example, the first flavourant may comprise one or more of water, ethanol, propylene glycol, and natural oils. In some examples, the first flavourant is in the form of an oil, a mixture, an emulsion, an organic solution, or an aqueous solution.

The first flavourant is contained in an amount of between 0.1 wt% and 20 wt% based on the total weight of the content. In some examples, the first flavourant is contained in an amount of between 0.1 wt% and 18 wt%, between 0.1 wt% and 15 wt%, between 0.1 wt% and 14 wt%, between 0.1 wt% and 12.5 wt%, between 0.1 wt% and 10 wt%, between 0.5 wt% and 10 wt%, between 1 wt% and 10 wt%, between 1 wt% and 7.5 wt%, between 1 wt% and 5 wt%, between 2 wt% and 5 wt%, between 2.5 wt% and 4.5 wt%, between 2.5 wt% and 4 wt%, or between 3 wt% and 4 wt%, based on the total weight of the content.

In some examples, the first flavourant is contained in an amount of 0.1 wt% or more, 0.25 wt% or more, 0.5 wt% or more, 0.75 wt% or more, 1 wt% or more, 1.25 wt% or more, 1.5 wt% or more, 1.75 wt% or more, 2 wt% or more, 2.25 wt% or more, 2.5 wt% or more, 2.75 wt% or more, 3 wt% or more, 3.1 wt% or more, 3.2 wt% or more, 3.25 wt% or more, 3.5 wt% or more, 3.75 wt% or more, 4 wt% or more, 4.5 wt% or more, 5 wt% or more, 5.5 wt% or more, 6 wt% or more, 6.5 wt% or more, 7 wt% or more, 7.5 wt% or more, 8 wt% or more, 8.5 wt% or more, 9 wt% or more, 9.5 wt% or more, 10 wt% or more, 11 wt% or more, 12 wt% or more, 12.5 wt% or more, or 15 wt% or more, based on the total weight of the content. In some examples, the first flavourant is contained in an amount of 2 wt% or more based on the total weight of the content. In some examples, the first flavourant is contained in an amount of 3 wt% or more based on the total weight of the content.

In some examples, the first flavourant is contained in an amount of 20 wt% or less, 15 wt% or less, 14 wt% or less, 13 wt% or less, 12 wt% or less, 11 wt% or less, 10 wt% or less, 9.5 wt% or less, 9 wt% or less, 8.5 wt% or less, 8 wt% or less, 7.5 wt% or less, 7 wt% or less, 6.5 wt% or less, 6 wt% or less, 5.75 wt% or less, 5.5 wt% or less, 5.25 wt% or less, 5 wt% or less, 4.75 wt% or less, 4.5 wt% or less, 4.25 wt% or less, 4 wt% or less, 3.75 wt% or less, 3.5 wt% or less, 3.4 wt% or less, 3.3 wt% or less, 3.2 wt% or less, 3.15 wt% or less, 3.1 wt% or less, 3 wt% or less, 2.75 wt% or less, 2.5 wt% or less, 2.25 wt% or less, 2 wt% or less, 1.75 wt% or less, 1.5 wt% or less, 1.25 wt% or less, 1 wt% or less, 0.75 wt% or less, or 0.5 wt% or less, based on the total weight of the content. In some examples, the first flavourant is contained in an amount of 5 wt% or less based on the total weight of the content. In some examples, the first flavourant is contained in an amount of 4 wt% or less based on the total weight of the content.

The flavourant may be evenly dispersed throughout the contents or may be provided in isolated locations and/or varying concentrations throughout the contents. In some examples, the first flavourant may be loaded onto fibres and/or particles contained in the content. For example, the first flavourant may be loaded onto fibres and/or particles prior to forming the content. In some examples, the first flavourant may be mixed evenly within the content.

Also described herein, is the use of a first flavourant comprising eucalyptus flavourant in a smokeless article to improve the user experience. In some examples, the use is to increase the "freshness" of the article. In some examples, the use is to provide a cooling sensation to the user. In some examples, the use is to provide a soothing sensation to the user.

**A second flavourant** which comprises or consists of a non-eucalyptus flavour component is included in the content. The second flavourant does not comprise a eucalyptus flavour component. In some examples, the second flavourant consists of a non-eucalyptus flavour component. For the avoidance of doubt, the second flavourant does not comprise natural eucalyptus, synthetic eucalyptus, or artificial eucalyptus.

A non-eucalyptus flavour component (which may also be referred to as a non-eucalyptus flavourant) is one which does not provide a flavour and/or aroma associated with eucalyptus. For example, a non-eucalyptus flavour component does not contain a botanical of *Eucalyptus,* such as eucalyptol. For example, a non-eucalyptus flavour component does not contain natural eucalyptus or synthetic/artificial eucalyptus. For example, a non-eucalyptus flavour component does not contain a synthetic compound providing the flavour and/or aroma of eucalyptus.

In some examples, the second flavourant is menthol or comprises a flavour component providing a flavour selected from the group consisting of rosemary, peppermint, frankincense, mint, spearmint, wintergreen, tea tree oil, citrus, oregano, bitter orange, grapefruit, sweet orange, green tea, violet, frankincense, anise, apple, apricot, banana, berries, guava, grape, melon, papaya, peach, pear, peas, clove, ginger, mustard, nuts, honey, coconut, passion fruit, olive, avocado, plum, rose apple, starfruit, sesame seed, tamarind, fig, cardamom, coriander seed and leaf, quince, lychee, dill, lovage, kiwi, loquat, sage, Chinese quince, chilli, nutmeg, cinnamon, lavender, basil, cocoa, vanilla, spice, star anise, fennel, and tobacco. In some examples, the second flavourant is menthol or comprises a flavour component providing a flavour selected from the group consisting of rosemary, peppermint, frankincense, spearmint, wintergreen, tea tree oil, citrus, and oregano. In some examples, the second flavourant is menthol or comprises a flavour component providing a flavour selected from peppermint, spearmint, and wintergreen. In some examples, the second flavourant is menthol.

In some examples, the second flavourant comprises or consists of a mint flavour component (i.e. a flavourant which provides a mint flavour, including the flavour of menthol, spearmint and peppermint).

In some examples, the second flavourant comprises or consists of one or more of a mint botanical, menthol (e.g. L-menthol), or a menthol derivative. In some examples, the second flavourant comprises peppermint or spearmint. In some examples, the second flavourant comprises or consists of menthol or a menthol derivative such as menthyl lactate or menthoxypropanediol. In some examples, the second flavourant comprises a constituent, derivative, or extract of a plant of the *Mentha* genus. In some examples, the second flavourant comprises a mint botanical. In this way, the cooling sensation in the user's mouth may be enhanced, for example by dual action on the TRPM8 protein. Additionally, an oral product having a mint or menthol flavour component as well as a eucalyptus flavourant and an additional cooling agent can result in a substantially greater cooling and soothing sensation, thus amplifying the user's pleasurable experience. In some examples, the first flavourant comprises eucalyptus flavourant and menthol, and the second flavourant is menthol (acting as supplemental menthol). In this way, a greater cooling and soothing effect can be achieved, especially in combination with the additional non-eucalyptus, non-menthol cooling agent. This ensures a maximum number of TRPM8 proteins can be activated in the user's mouth.

In some examples, the second flavourant does not comprise mint. In some examples, the second flavourant does not comprise peppermint or spearmint. In some examples, the second flavourant does not contain a constituent, derivative, or extract of a plant of the *Mentha* genus. In some examples, the second flavourant does not contain a mint botanical. In some examples, the second flavourant does not contain menthol. In this way, the second flavourant may be one which does not activate the TRPM8 protein. Since this protein is activated by eucalyptol, in some examples it may be desirable for the second flavourant to act in a different way in order to provide a unique flavour and organoleptic experience. In some examples, the second flavourant does not comprise a TRPM8 agonist.

The second flavourant may be or comprise a natural flavourant, a synthetic flavourant, or a mixture thereof. "Natural flavourant" and "synthetic flavourant" may be defined analogously to "natural eucalyptus" and "synthetic eucalyptus", respectively, defined above. A natural flavourant is obtained from natural sources without chemical processing (though mechanical processing may be used). A synthetic flavourant is obtained by chemical synthesis. For the avoidance of doubt, a synthetic flavourant may include a compound which is made synthetically but is otherwise available naturally. Additionally, a synthetic flavourant may use natural precursors or starting materials.

The second flavourant may comprise one or more botanicals which is not eucalyptus. In other words, in some examples, the second flavourant comprises one or more constituent, derivative, extract, or a mixture thereof, obtained from, or which can otherwise be found in, a plant which is not of the *Eucalyptus* genus. In other words, when the second flavourant comprises a botanical, the botanical is not obtained from a *Eucalyptus* plant, i.e. it is a non-eucalyptus botanical. The terms "constituent", "derivative", and "extract", in reference to a plant other than *Eucalyptus* take the same meaning as described herein with reference to *Eucalyptus.* Similarly, with reference to any other plant there may be "natural", "synthetic", and/or "artificial" flavourants, defined in an analogous manner to natural, synthetic, and artificial eucalyptus.

Suitable botanicals include, but are not limited to, rosemary, peppermint, frankincense, menthol, spearmint, wintergreen, tea tree oil, citrus, oregano, bitter orange, grapefruit, sweet orange, green tea, violet, frankincense, anise, apple, apricot, banana, berries, guava, grape, melon, papaya, peach, pear, peas, clove, ginger, mustard, nuts, honey, coconut, passion fruit, olive, avocado, plum, rose apple, starfruit, sesame seed, tamarind, fig, cardamom, coriander seed and leaf, quince, lychee, dill, lovage, kiwi, loquat, sage, Chinese quince, chilli, nutmeg, cinnamon, lavender, basil, cocoa, vanilla, spice, star anise, fennel, and tobacco. For example, the second flavourant comprises an oil of one or more of said botanicals. In some examples, the second flavourant does not comprise peppermint, spearmint, or menthol.

In some examples, the second flavourant does not comprise a liquorice flavourant. As explained with respect to the first flavourant, the presence of a liquorice flavourant may lead to unwanted numbing/dulling which could reduce the cooling effect of the product.

In some examples, the second flavourant comprises a botanical which is a source of 1-octanol (i.e., the botanical is obtained from a plant in which 1-octanol is naturally found), such as bitter orange, grapefruit, sweet orange, green tea, violet, frankincense, apple, apricot, banana, citrus peel oils and juices, berries, guava, grape, melon, papaya, peach, pear, peas, clove, ginger, mustard, spearmint oil, nuts, honey, coconut, passion fruit, olive, avocado, plum, rose apple, starfruit, sesame seed, tamarind, fig, cardamom, coriander seed and leaf, quince, lychee, dill, lovage, kiwi, loquat, sage, and Chinese quince.

In some examples, the second flavourant possesses cooling and/or soothing properties. In some examples, the second flavourant comprises one or more selected from rosemary, peppermint, frankincense, menthol, spearmint, wintergreen, tea tree, citrus, and oregano. In some examples, the second flavourant comprises a constituent, derivative, or extract of one or more of *Salvia rosmarinus, Mentha* × *piperita, Boswellia sacra, Mentha spicata, Gaultheria procumbens, Camellia sinensis, Citrus hystrix, Citrus macroptera, Citrus maxima, Citrus medica,* and *Origanum vulgare,* or of any hybrid of said species. In this way, the cooing and/or soothing effect of the product can be enhanced compared to a product only comprising eucalyptus as the sole flavourant.

In some examples, the second flavourant comprises an essential oil. An essential oil may be interpreted as being a flavour component which provides a flavour of the plant from which the oil is obtained.

In some examples, the second flavourant comprises a cooling essential oil, for example selected from: German chamomile oil (*Matricaria rectutita*), geranium oil (*Pelargonium graveolens var roseum*), lavender oil (*Lavandula angustifolia*), lime oil (*Citrus aurantifolia*), orange (sweet) oil (*Citrus sinensis*), palmarosa oil (*Cymbopogon martinii var. motia*), sandalwood oil (*Santalum album*), and vetiver oil (*Vetiveria zizanoides*). A cooling essential oil provides a cooling sensation in a user's mouth. By using a cooling essential oil, the freshness and cooling sensation of eucalyptus can be enhanced and prolonged.

In some examples, the second flavourant comprises a warming essential oil, for example selected from: black pepper oil (*Piper nigrum*), cinnamon bark oil (*Cinnamomum zeylanicum*), cardamom oil (*Elettaria cardamomum*), clove bud oil (*Eugenia caryophyllata*), coriander oil (*Coriandrum sativum*), ginger oil (*Zingiber officinale*), gingergrass oil (*Cymbopogon martinii var. Sofia*), juniper berry oil (*Juniperus communis*), marjoram (sweet) oil (*Origanum majorana*), and myrrh oil (*Commiphora myrrha*). A warming essential oil provides a warming sensation in a user's mouth. By using a warming essential oil, a unique flavour profile and organoleptic experience can be achieved through the contrast with the cooling eucalyptus, thus heightening the user's experience and perception.

In some examples, the second flavourant may be selected for its cooling, tingling, warming, or numbing sensation. In some examples, the second flavourant is selected for its pleasant taste. In some examples, the second flavourant is selected for its pleasant aroma. In some examples, the second flavourant is selected for its sweet, sour, or spicy taste.

In some examples, the second flavourant comprises a plant constituent or derivative. In some examples, the second flavourant comprises a compound which has been isolated from a plant. In some examples, the second flavourant comprises camphor. In some examples, the second flavourant comprises menthol.

In some examples, the second flavourant comprises a single botanical. In some examples, the flavourant comprises a botanical of only one plant species or hybrid. In some examples, the second flavourant comprises a constituent, derivative, or extract of only one plant species or hybrid. In that way, the flavour profile can be accurately tuned between eucalyptus and one other botanical.

In some examples, the second flavourant comprises two or more botanicals. In some examples, the second flavourant comprises a constituent, derivative, or extract of two or more plant species or hybrids. In that way, the flavour profile can be expanded further, and the user experience can be enhanced, for example through additional flavours, sensations, and aromas.

The second flavourant is contained in an amount of between 0.1 wt% and 20 wt% based on the total weight of the content. In some examples, the second flavourant is contained in an amount of between 0.1 wt% and 18 wt%, between 0.1 wt% and 15 wt%, between 0.1 wt% and 14 wt%, between 0.1 wt% and 12.5 wt%, between 0.1 wt% and 10 wt%, between 0.5 wt% and 10 wt%, between 1 wt% and 10 wt%, between 1 wt% and 7.5 wt%, between 1 wt% and 5 wt%, between 2 wt% and 5 wt%, between 2.5 wt% and 4.5 wt%, between 2.5 wt% and 4 wt%, between 3 wt% and 4 wt%, between 2.5 wt% and 10 wt%, between 2.5 wt% and 7.5 wt%, between 4 wt% and 8 wt%, between 4 wt% and 7 wt%, between 4 wt% and 6 wt%, between 4 wt% and 5 wt%, between 4.5 wt% and 5.5 wt%, or between 5 wt% and 6 wt%, based on the total weight of the content.

In some examples, the second flavourant is contained in an amount of 0.1 wt% or more, 0.25 wt% or more, 0.5 wt% or more, 0.75 wt% or more, 1 wt% or more, 1.25 wt% or more, 1.5 wt% or more, 1.75 wt% or more, 2 wt% or more, 2.25 wt% or more, 2.5 wt% or more, 2.75 wt% or more, 3 wt% or more, 3.1 wt% or more, 3.2 wt% or more, 3.25 wt% or more, 3.5 wt% or more, 3.75 wt% or more, 4 wt% or more, 4.5 wt% or more, 5 wt% or more, 5.5 wt% or more, 6 wt% or more, 6.5 wt% or more, 7 wt% or more, 7.5 wt% or more, 8 wt% or more, 8.5 wt% or more, 9 wt% or more, 9.5 wt% or more, 10 wt% or more, 11 wt% or more, 12 wt% or more, 12.5 wt% or more, or 15 wt% or more, based on the total weight of the content. In some examples, the second flavourant is contained in an amount of 2 wt% or more based on the total weight of the content. In some examples, the second flavourant is contained in an amount of 3 wt% or more based on the total weight of the content.

In some examples, the second flavourant is contained in an amount of 20 wt% or less, 15 wt% or less, 14 wt% or less, 13 wt% or less, 12 wt% or less, 11 wt% or less, 10 wt% or less, 9.5 wt% or less, 9 wt% or less, 8.5 wt% or less, 8 wt% or less, 7.5 wt% or less, 7 wt% or less, 6.5 wt% or less, 6 wt% or less, 5.75 wt% or less, 5.5 wt% or less, 5.25 wt% or less, 5 wt% or less, 4.75 wt% or less, 4.5 wt% or less, 4.25 wt% or less, 4 wt% or less, 3.75 wt% or less, 3.5 wt% or less, 3.4 wt% or less, 3.3 wt% or less, 3.2 wt% or less, 3.15 wt% or less, 3.1 wt% or less, 3 wt% or less, 2.75 wt% or less, 2.5 wt% or less, 2.25 wt% or less, 2 wt% or less, 1.75 wt% or less, 1.5 wt% or less, 1.25 wt% or less, 1 wt% or less, 0.75 wt% or less, or 0.5 wt% or less, based on the total weight of the content. In some examples, the second flavourant is contained in an amount of 5 wt% or less based on the total weight of the content. In some examples, the second flavourant is contained in an amount of 4 wt% or less based on the total weight of the content.

In some examples, the flavourant is provided in solid form. In some examples, the flavourant is provided in liquid form. In some examples, the flavourant is provided as a solution. In some examples, the flavourant is provided as a gel. In some examples, the flavourant is provided as an oil. In some examples, the flavourant consists of a single compound (e.g. menthol, camphor). In some examples, the flavourant is adhered to a solid substrate.

In some examples, the second flavourant consists of one or more botanicals. In other words, in some examples, the second flavourant consists only of one or more plant constituents, derivatives, extracts, or mixtures thereof, such as a plant oil.

In some examples, the second flavourant, similarly to the first flavourant, may additionally comprise one or more excipients, diluents, or solvents. Suitable excipients, diluents, and solvent for flavour compositions are known to those skilled in the art. For example, the second flavourant may comprise one or more of water, ethanol, propylene glycol, and natural oils. In some examples, the second flavourant is in the form of an oil, a mixture, an emulsion, an organic solution, or an aqueous solution.

Also described herein, is the use of a second flavourant comprising a non-eucalyptus flavourant, in combination with a first flavourant comprising eucalyptus flavourant, in a smokeless article to improve the user experience. In some examples, the use is to increase the "freshness" of the article. In some examples, the use is to increase the "freshness" of the article, relative to an article comprising only the first flavourant. In some examples, the use is to provide a cooling sensation to the user. In some examples, the use is to provide an enhanced cooling sensation to the user, relative to an article comprising only the first flavourant. In some examples, the use is to provide a soothing sensation to the user. In some examples, the use is to provide an enhanced soothing sensation to the user, relative to an article comprising only the first flavourant. In some examples, the use is to provide a broader flavour profile compared to an article comprising only one flavourant. In some examples, the use is to provide a broader aroma profile compared to an article comprising only one flavourant. In some examples, the use is to provide a longer-lasting flavour or aroma profile compared to an article comprising only one flavourant.

Also described herein, is a smokeless article wherein the first flavourant is natural eucalyptus and the second flavourant is synthetic eucalyptus (such as artificial eucalyptus). Other features of said smokeless article (e.g., amounts, ratios, cooling agent, fibrous material, additives, etc.) may be as defined according to the first aspect.

**A cooling agent** distinct from eucalyptol and menthol is included in the content of the smokeless article. A cooling agent is a substance that creates a sensation of coolness or freshness on the skin, mucous membranes, or inside the mouth, for example by interacting with sensory receptors (such as TRPM8) that respond to cold stimuli. These agents achieve this effect without necessarily lowering the temperature of the area, and provide refreshing and soothing properties. The cooling agent preferably provides a cooling sensation inside the mouth when the smokeless article is consumed.

The cooling agent is distinct from eucalyptol and menthol. That is, the cooling agent is not and does not comprise eucalyptol or menthol. In some examples, the cooling agent does not comprise a eucalyptus compound.

In some examples, the cooling agent comprises or consists of one or more selected from: WS-3, WS-5, WS-23, WS-12, icilin, menthyl lactate, menthoxypropanediol, isopulegol, camphor, borneol, isoborneol, thymol, carvone, and a pentose monosaccharide (e.g. xylitol). In some examples, the cooling agent comprises or consists of one or more selected from: WS-3, WS-5, WS-23, WS-12, icilin, camphor, borneol, isoborneol, thymol, and carvone.

In some examples, the cooling agent comprises a pentose monosaccharide as defined herein. For example, the pentose monosaccharide may be arabitol, ribitol, or xylitol. In some examples, the cooling agent does not comprise a pentose monosaccharide.

In some examples, the cooling agent comprises two or more non-eucalyptol, non-menthol cooling agents. In some examples, the cooling agent comprises xylitol and one or more of WS-3, WS-5, WS-23, WS-12, icilin, isopulegol, camphor, borneol, isoborneol, thymol, or carvone. In some examples, the cooling agent comprises xylitol and one or more of WS-3, WS-5, WS-23, or WS-12. In some examples, the cooling agent comprises xylitol and WS-5.

In some examples, the cooling agent is synthetic. Synthetic cooling agents include: WS-3, WS-5, WS-23, WS-12, icilin, isopulegol, menthyl lactate, and menthoxypropanediol. In some examples, the cooling agent is natural. Natural cooling agents include: isopulegol, camphor, borneol, isoborneol, thymol, and carvone. In some examples, the cooling agent is a monoterpene. Some cooling agents are found naturally but may be obtained synthetically, such as isopulegol. In some examples, the cooling agent is not found naturally. In some examples, the cooling agent does not comprise isopulegol.

WS-3 (N-ethyl-p-menthane-3-carboxamide; CAS no. 39711-79-0) provides a moderate cooling effect with a smooth, long-lasting sensation. WS-5 (N-((ethoxycarbonyl)methyl)-p-menthane-3-carboxamide; CAS no. 68489-14-5) is a stronger cooling agent than WS-3, with a more intense and long-lasting cooling sensation. WS-12 ((1R*,2S*)-N-(4-Methoxyphenyl)-5-methyl-2-(1-methylethyl)cyclohexanecarboxamide; CAS no. 68489-09-8) is one of the most potent synthetic cooling agents, offering a powerful and prolonged effect. WS-23 (N,2,3-trimethyl-2-isopropyl butanamide; CAS no. 51115-67-4) provides a strong, immediate cooling sensation with minimal odor and taste. Icilin (CAS no. 36945-98-9) is a highly potent cooling agent that mimics menthol's effects by activating cold-sensitive TRPM8 receptors. Menthyl lactate is a mild, synthetic derivative of menthol, which may provide a more gentle cooling effect than others listed. Menthoxypropanediol is a synthetic menthol derivative which provides a long-lasting cooling effect. Isopulegol is a monoterpene alcohol with a minty, cooling aroma and flavour - it is a key intermediate in the biosynthesis of menthol but can be obtained synthetically. Such cooling agents are commercially available from commercial vendors such as MedChemExpress, Sigma Aldrich, and Tocris Bioscience.

Borneol is a monoterpene alcohol with a camphor-like odor, used for its cooling properties, found in camphor tree, rosemary, and other aromatic plants. Isoborneol is a stereoisomer of borneol and is also a monoterpene alcohol, with a slightly milder camphoraceous scent and cooling effect - it is found in camphor, rosemary, and certain essential oils. Thymol is a monoterpene phenol with a warm, spicy aroma, with mild cooling effects - it is found in thyme, oregano, and other mint-family plants. Carvone is a monoterpene ketone with a minty or caraway-like scent, depending on its enantiomer, and with a mild cooling effect - it is found in spearmint (I(-)-carvone) and caraway seeds (d(+)-carvone).

An approximate ranking of certain cooling agents, from strongest cooling effect to weakest is: icilin; WS-12; WS-23; WS-5; menthol; WS-3; menthoxypropanediol; menthyl lactate; camphor; eucalyptol (1,8-cineole); borneol; isoborneol; thymol; carvone.

In some examples, the cooling agent has a stronger cooling effect than eucalyptus. In some examples, the cooling agent has a stronger cooling effect than menthol. In some examples, the cooling agent comprises or consists of WS-5. In this way, it is possible to provide a product with a stronger cooling effect than is ordinarily possible for products comprising eucalyptus and/or menthol as the only cooling components. Therefore, an enhanced cooling and soothing experience can be provided to the user, providing the feeling of mouth freshness. For example, a smokeless article comprising a eucalyptus flavourant, menthol (e.g. as a second flavourant), and a cooling agent stronger than eucalyptus or menthol can provide a cooling and soothing effect which is greater than an article comprising only eucalyptus and/or menthol as a cooling agent. Therefore, such an article will result in an enhanced user experience and a greater perception of mouth freshness.

In some examples, the cooling agent is contained in an amount of between 0.01 wt% and 5 wt% based on the total weight of the content. In some examples, the first flavourant is contained in an amount of between 0.01 wt% and 4 wt%, between 0.01 wt% and 3 wt%, between 0.01 wt% and 2 wt%, between 0.01 wt% and 1 wt%, between 0.05 wt% and 4 wt%, between 0.05 wt% and 3.5 wt%, between 0.05 wt% and 3 wt%, between 0.08 wt% and 3 wt%, between 0.1 wt% and 3 wt%, between 0.1 wt% and 2.5 wt%, between 0.1 wt% and 2 wt%, between 0.1 wt% and 1.5 wt%, between 0.1 wt% and 1 wt%, between 0.1 wt% and 0.8 wt%, between 0.2 wt% and 0.7 wt%, between 0.2 wt% and 0.6 wt%, between 0.3 wt% and 0.6 wt%, between 0.4 wt% and 0.6 wt%, between 0.2 wt% and 0.5 wt%, between 0.2 wt% and 0.4 wt%, based on the total weight of the content. In some examples, the cooling agent is contained in an amount of between 0.1 wt% and 1 wt%, such as between 0.2 wt% and 0.6 wt%.

In some examples, the cooling agent is contained in an amount of 0.01 wt% or more, 0.02 wt% or more, 0.03 wt% or more, 0.04 wt% or more, 0.05 wt% or more, 0.06 wt% or more, 0.07 wt% or more, 0.08 wt% or more, 0.09 wt% or more, 0.1 wt% or more, 0.2 wt% or more, 0.3 wt% or more, 0.4 wt% or more, 0.5 wt% or more, 0.6 wt% or more, 0.7 wt% or more, 0.8 wt% or more, 0.9 wt% or more, 1 wt% or more, 1.25 wt% or more, 1.5 wt% or more, 1.75 wt% or more, or 2 wt% or more. For example, it is contained in an amount of 0.05 wt% or more, such as 0.1 wt% or more. In some examples, the cooling agent is contained in an amount of 0.2 wt% or more.

In some examples, the cooling agent is contained in an amount of 5 wt% or less, 4.5 wt% or less, 4 wt% or less, 3.5 wt% or less, 3 wt% or less, 2.75 wt% or less, 2.5 wt% or less, 2.25 wt% or less, 2 wt% or less, 1.75 wt% or less, 1.5 wt% or less, 1.25 wt% or less, 1 wt% or less, 0.9 wt% or less, 0.8 wt% or less, 0.7 wt% or less, 0.6 wt% or less, 0.5 wt% or less, 0.4 wt% or less, 0.3 wt% or less, 0.2 wt% or less, 0.1 wt% or less, 0.09 wt% or less, 0.08 wt% or less, 0.07 wt% or less, 0.06 wt% or less, or 0.05 wt% or less. For example, it is contained in an amount of 2wt% or less, 1.5 wt% or less, such as 1 wt% or less. In some examples, the cooling agent is contained in an amount of 0.6 wt% or less.

Also described herein is a smokeless article comprising a first and second flavourant as described herein, but which does not contain the cooling agent described herein. The other features of said described smokeless article (e.g., the content and its components) may be as defined herein.

**A pentose monosaccharide** may be included in the content. Without wishing to be bound by theory, it is thought that, when included as part of a content, pentose monosaccharides can enhance cooling and soothing sensations by chemical interaction with a user. That is, pentose monosaccharides are thought to increase cooling and soothing sensations beyond sensations by mental association.

A pentose monosaccharide may be included in addition to the cooling agent described herein (e.g., the cooling agent does not comprise a pentose monosaccharide). Alternatively, the pentose monosaccharide may be included as, or as part of, the cooling agent described herein.

When a first flavourant is synthetic eucalyptus flavourant and a pentose monosaccharide is included in the content, the content may exhibit properties typically exhibited by providing a natural eucalyptus flavourant, thereby contributing to cooling and soothing sensations by chemical interaction during use without the need for natural eucalyptus components. In other words, the combination of a pentose monosaccharide and synthetic eucalyptus flavourant may simulate natural eucalyptus components (such as eucalyptus botanicals) beyond flavour alone, to provide cooling and/or soothing sensations during use, with may be of a similar intensity or enhanced intensity compared to natural eucalyptus flavourants.

Examples of suitable pentose monosaccharides include but are not limited to arabitol, ribitol, and xylitol. The term arabitol may refer to D-arabitol, L-arabitol, or a mixture thereof. Correspondingly, ribitol may refer to a D- or L-stereoisomer of ribitol, or a mixture thereof. Xylitol refers exclusively to the compound otherwise known as (2R,3R,4S)-pentane-1,2,3,4,5-pentol.

In some examples, the content comprises a pentose monosaccharide selected from one or more of arabitol, ribitol, and xylitol. In some cases, the pentose monosaccharide is xylitol.

An amount of pentose monosaccharide is not particularly limited, and it is thought that by increasing an amount of pentose monosaccharide, soothing and cooling sensations can be increased.

In some examples, the pentose monosaccharide (e.g., xylitol) is present in an amount of between 0.1 wt% and 10 wt%, for example between 0.1 wt% and 8 wt%, for example between 0.1 wt% and 6 wt%, for example between 0.1 wt% and 4 wt%, for example between 0.2 wt% and 4 wt%, for example between 0.5 wt% and 4 wt%, for example between 1 wt% and 4 wt%, for example between 2 wt% and 4 wt%, for example between2 wt% and 3 wt%, between 2.5 wt% and 3.5 wt%, or between 3 wt% and 4 wt%, based on the total weight of the content. In some examples, the amount of the pentose monosaccharide (e.g., xylitol) is about 3 wt%.

In some examples, the pentose monosaccharide is present in an amount of between 0.1 wt% and 8 wt% based on the total weight of the content, wherein the pentose monosaccharide is one selected from the group consisting of arabitol, rabitol, and xylitol.

In some examples, the pentose monosaccharide (e.g., xylitol) is present in an amount of 10 wt% or less, for example 8 wt% or less, for example 6 wt% or less, for example 5 wt% or less, for example 4 wt% or less, for example 3 wt% or less, for example 2 wt% or less, or for example 1 wt% or less, based on the total weight of the content. In some examples, the amount is 3 wt% or less.

**A first ratio** of the weight of the second flavourant to the first flavourant within the content may be defined, i.e. the weight ratio of the second flavourant to the first flavourant. For example, the first ratio may be defined as the amount by weight of the second flavourant in the content divided by the amount by weight of the first flavourant in the content, i.e. the first ratio is defined as [amount by weight of second flavourant in content] / [amount by weight of first flavourant in content].

In some examples, the first ratio is between 0.01 and 100. In some examples, the first ratio is between 0.05 and 75, between 0.05 and 50, between 0.05 and 25, between 0.075 and 25, between 0.075 and 20, between 0.075 and 15, between 0.1 and 15, between 0.1 and 12.5, between 0.1 and 10, between 0.1 and 7.5, between 0.1 and 5, between 0.1 and 2.5, between 0.1 and 2, between 0.2 and 10, between 0.25 and 7.5, between 0.25 and 5, between 0.25 and 2.5, between 0.25 and 1.5, between 0.25 and 1, or between 0.5 and 1.

In some examples, the first ratio is between 0.1 and 10. In some examples, the first ratio is between 0.1 and 2.

In some examples, the first ratio is 0.01 or more, 0.05 or more, 0.1 or more, 0.2 or more, 0.25 or more, 0.3 or more, 0.4 or more, 0.5 or more, 0.6 or more, 0.7 or more, 0.75 or more, 0.8 or more, 0.9 or more, 1 or more, 1.5 or more, 2 or more, 2.5 or more, 3 or more, 4 or more, 5 or more, 7.5 or more, 10 or more, 12.5 or more, 15 or more, 17.5 or more, 20 or more, 22.5 or more, 25 or more, 30 or more, 35 or more, 40 or more, 45 or more, 50 or more, 60 or more, 75 or more, 80 or more, or 90 or more. In some examples, the first ratio is 100 or less, 90 or less, 80 or less, 75 or less, 60 or less, 50 or less, 45 or less, 40 or less, 35 or less, 30 or less, 27.5 or less, 25 or less, 22.5 or less, 20 or less, 17.5 or less, 15 or less, 12.5 or less, 10 or less, 7.5 or less, 5 or less, 4 or less, 3 or less, 2.5 or less, 2 or less, 1.5 or less, 1.25 or less, 1 or less, 0.9 or less, 0.8 or less, 0.75 or less, 0.7 or less, 0.6 or less, 0.5 or less, 0.4 or less, 0.3 or less, 0.25 or less, 0.2 or less, 0.1 or less, or 0.05 or less.

**A second ratio** may also be defined between the weight of the cooling agent and the first flavourant, i.e. the weight ratio of the cooling agent to the first flavourant. For example, the second ratio may be defined as the amount by weight of the cooling agent in the content divided by the amount by weight of the first flavourant in the content, i.e. the second ratio is defined as [amount by weight of cooling agent in content] / [amount by weight of first flavourant in content].

In some examples, the second ratio is between 0.01 and 10. In some examples, the second ratio is between 0.05 and 1. In some examples, the second ratio is between 0.1 and 0.5.

In some examples, the second ratio is between 0.01 and 100. In some examples, the second ratio is between 0.05 and 75, between 0.05 and 50, between 0.05 and 25, between 0.075 and 25, between 0.075 and 20, between 0.075 and 15, between 0.1 and 15, between 0.1 and 12.5, between 0.1 and 10, between 0.1 and 7.5, between 0.1 and 5, between 0.1 and 2.5, between 0.1 and 2, between 0.2 and 10, between 0.25 and 7.5, between 0.25 and 5, between 0.25 and 2.5, between 0.25 and 1.5, between 0.25 and 1, or between 0.5 and 1.

In some examples, the second ratio is 0.01 or more, 0.02 or more, 0.03 or more, 0.04 or more, 0.05 or more, 0.06 or more, 0.07 or more, 0.08 or more, 0.09 or more, 0.1 or more, 0.125 or more, 0.15 or more, 0.2 or more, 0.25 or more, 0.3 or more, 0.4 or more, 0.5 or more, 0.6 or more, 0.7 or more, 0.8 or more, 0.9 or more, or 1 or more. In some examples, the second ratio is 10 or less, 9 or less, 8 or less, 7 or less, 6 or less, 5 or less, 4 or less, 3 or less, 2.5 or less, 2 or less, 1.5 or less, 1.25 or less, 1 or less, 0.9 or less, 0.8 or less, 0.7 or less, 0.6 or less, 0.5 or less, 0.4 or less, 0.3 or less, 0.25 or less, 0.2 or less, 0.15 or less, 0.125 or less, 0.1 or less, 0.09 or less, 0.08 or less, 0.07 or less, 0.06 or less, or 0.05 or less.

**A third ratio** may also be defined between the weight of the cooling agent and the second flavourant (e.g. menthol), i.e. the weight ratio of the cooling agent to the second flavourant. For example, the third ratio may be defined as the amount by weight of the cooling agent in the content divided by the amount by weight of the second flavourant in the content, i.e. the third ratio is defined as [amount by weight of cooling agent in content] / [amount by weight of second flavourant in content]. The third ratio may be defined in the same way as the second ratio, above.

**A third flavourant** may be included in the content of some examples. The third flavourant may be defined in the same manner as the second flavourant, for example with respect to type of flavour component or botanical, and the amount in which it is contained. The third flavourant, when included, comprises or consists of a flavour component (e.g. a botanical) or compound different to that of the first and second flavourant.

In some examples, the third flavourant comprises a eucalyptus flavourant, as defined with respect to the first flavourant. For example, the third flavourant may comprise an artificial eucalyptus flavourant. For example, the first flavourant may be natural eucalyptus, the second flavourant menthol, and the third flavourant artificial eucalyptus. In this way, a smokeless article is provided which provides a strong cooling effect and a prominent eucalyptus flavour which establishes a mental association with cooling and soothing sensations in the user. This is further enhanced by the additional cooling agent, such as WS-5.

In some examples, the third flavourant does not comprise a eucalyptus flavourant. For example, the third flavourant is defined in accordance with the second flavourant (but the second and third flavourants are different, such as providing a different flavour).

In addition, a fourth flavourant, a fifth flavourant, or an even further flavourant may be included in the content, each comprising or consisting of a flavour component (e.g. a botanical) different to that of any preceding flavourant. Such additional flavourants may be defined in the same manner as the second flavourant, for example with respect to type of flavour component or botanical and the amount in which it is contained.

In some examples, the third flavourant does not comprise menthol or a mint flavourant. In some examples, the second flavourant comprises menthol or a mint flavourant, and the third flavourant does not comprise menthol or a mint flavourant.

In some examples, none of the flavourants comprise a mint flavourant (e.g. a mint botanical), such as peppermint or spearmint. In some examples, none of the flavourants comprise menthol. In some examples, the content is substantially free of menthol. In some examples, the content is free of menthol. In some examples, the content does not contain a constituent, derivative, or extract of a plant of the *Mentha* genus.

In some examples, none of the flavourants comprise a liquorice flavourant (e.g., a liquorice botanical), such as European liquorice, American liquorice, Chinese liquorice, black liquorice, or red liquorice. In some examples, none of the flavourants comprise liquorice. In some examples, the content is substantially free of liquorice. In some examples, the content is free of liquorice. In some examples, the content does not contain a constituent, derivative, or extract of a plant of the *Glycyrrhiza* genus.

**The total amount of flavourant** is defined as the combined amount of the first flavourant and the second flavourant, and any additional (e.g., third, fourth, fifth, etc.) flavourant which may be present. In other words, the total amount of flavourant is the amount by weight of all flavourants, relative to the total weight of the content. In examples where there is only a first and second flavourant (i.e., not third or further flavourant), the total amount of flavourant is the amount of first flavourant plus the amount of second flavourant, by weight relative to the total weight of the content.

Unless specified otherwise, the amount of the cooling agent is not included in the total amount of the flavourant.

In some examples, the total amount of flavourant is between 0.1 wt% and 30 wt% based on the total weight of the content. In some examples, the total amount of flavourant is between 0.1 wt% and 25 wt%, 0.1 wt% and 20 wt%, between 0.1 wt% and 15 wt%, between 0.1 wt% and 12.5 wt%, between 0.1 wt% and 10 wt%, between 0.5 wt% and 10 wt%, between 1 wt% and 10 wt%, between 1 wt% and 7.5 wt%, between 1 wt% and 5 wt%, between 2 wt% and 5 wt%, between 2.5 wt% and 4.5 wt%, between 2.5 wt% and 4 wt%, or between 3 wt% and 4 wt%, based on the total weight of the content.

In some examples, the total amount of flavourant is 0.1 wt% or more, 0.25 wt% or more, 0.5 wt% or more, 0.75 wt% or more, 1 wt% or more, 1.25 wt% or more, 1.5 wt% or more, 1.75 wt% or more, 2 wt% or more, 2.25 wt% or more, 2.5 wt% or more, 2.75 wt% or more, 3 wt% or more, 3.1 wt% or more, 3.2 wt% or more, 3.25 wt% or more, 3.5 wt% or more, 3.75 wt% or more, 4 wt% or more, 4.5 wt% or more, 5 wt% or more, 5.5 wt% or more, 6 wt% or more, 6.5 wt% or more, 7 wt% or more, 7.5 wt% or more, 8 wt% or more, 8.5 wt% or more, 9 wt% or more, 9.5 wt% or more, 10 wt% or more, 11 wt% or more, 12 wt% or more, 13 wt% or more, 14 wt% or more, 15 wt% or more, 17.5 wt% or more, or 20 wt% or more, based on the total weight of the content. In some examples, the total amount of flavourant is 2 wt% or more based on the total weight of the content. In some examples, the total amount of flavourant is 3 wt% or more based on the total weight of the content.

In some examples, the total amount of flavourant is 25 wt% or less, 22.5 wt% or less, 20 wt% or less, 17.5 wt% or less, 15 wt% or less, 14 wt% or less, 13 wt% or less, 12 wt% or less, 11 wt% or less, 10 wt% or less, 9.5 wt% or less, 9 wt% or less, 8.5 wt% or less, 8 wt% or less, 7.5 wt% or less, 7 wt% or less, 6.5 wt% or less, 6 wt% or less, 5.75 wt% or less, 5.5 wt% or less, 5.25 wt% or less, 5 wt% or less, 4.75 wt% or less, 4.5 wt% or less, 4.25 wt% or less, 4 wt% or less, 3.75 wt% or less, 3.5 wt% or less, 3.4 wt% or less, 3.3 wt% or less, 3.2 wt% or less, 3.15 wt% or less, 3.1 wt% or less, 3 wt% or less, 2.75 wt% or less, 2.5 wt% or less, 2.25 wt% or less, 2 wt% or less, 1.75 wt% or less, 1.5 wt% or less, 1.25 wt% or less, 1 wt% or less, 0.75 wt% or less, or 0.5 wt% or less, based on the total weight of the content. In some examples, the total amount of flavourant is 5 wt% or less based on the total weight of the content. In some examples, the total amount of flavourant is 4 wt% or less based on the total weight of the content.

In some cases, the total amounts of flavourants also includes the amount of cooling agent and/or the amount of pentose monosaccharide (such as xylitol). For example, in some cases, the combined total amount of flavourants and pentose monosaccharide (e.g. xylitol) is between 0.1 wt% and 30 wt%; in some cases, the combined total amount of flavourants and cooling agent is between 0.1 wt% and 30 wt%; or in some cases, the combined total amount of flavourants, pentose monosaccharide (e.g. xylitol) and cooling agent is between 0.1 wt% and 30 wt%.

**Particles** may be included in the content of some examples of the smokeless articles according to the present disclosure. That is, in some examples, the content comprises a plurality of particles. The particles referred to herein are distinct from the fibrous material contained in the content. For example, the particles described herein may form a particulate component of the content, which is distinct from, though may be intimately mixed with, other components (e.g. the at least one fibrous material) of the content. In some examples, the particles may also be referred to as grains, granules, beads, microbeads, etc. In some examples, the nature of the particles may have an effect on the blend/powder characteristics of the content, and/or on the user experience of the product. In some examples, the particles may be wax particles, i.e. particles made from a wax, or including a wax.

In some examples, the particles are coloured particles, e.g. particles which have been coloured using one or more dyes or pigments. Such coloured particles may appear coloured to the human eye, reflecting a wavelength in the visible part of the electromagnetic spectrum. In some examples, the particles may be coloured wax particles, for example they may comprise one or more, typically one, selected from the group consisting of paraffin wax, Montan wax, carnauba wax, candelilla wax, beeswax, cottonseed wax, animal wax, microcrystalline wax, mineral wax, palm wax, rapeseed wax, and soy wax. In some examples, the particles are paraffin wax particles. In some examples, the particles are formed from uncoloured particles which have been mixed with a colourant. In some examples, the particles are formed from a coloured material (i.e., a material which has already been coloured by a colourant, or which is intrinsically coloured).

In some examples, the reflected light has a wavelength of between 380-750 nm. In some examples, the reflected light has a colour selected from red, yellow, green and blue. In some examples, the colour of the coloured particles is selected from: red, orange, yellow, green, blue, indigo, purple, violet, pink, brown, black, and grey. In some examples, the colour of the coloured particles is selected from: red, orange, yellow, green, blue, purple, violet, and black. In some examples, all of the coloured particles have the same colour. In some examples, some of the particles have a different colour to other particles.

In some examples, the coloured particles modify the colour of the content. In some examples, the coloured particles impart a speckled effect to the content.

In some examples, a suitable colour for the coloured particles is selected based on the intended experience of the user, such as flavour enhancement. For example, a green or blue colour may be chosen to enhance a eucalyptus experience, a red colour may be chosen to enhance a spice or heat experience, or a yellow colour may be chosen to enhance a citrus experience. The colour may be selected depending on the specific combination of first and second flavourant. In some examples, particles of two different colours may be selected to represent the two different flavourants. For example, particles of a first colour may be included to form a mental association with the first flavourant, and particles of a second colour may be included to form a mental association with the second flavour. It has been found that the user's perception of freshness and cooling is enhanced when a eucalyptus flavourant is combined with particles having a "cool" colour, such as blue, green, grey, or purple. This is enhanced even further when the second flavourant has a fresh, cooling, and/or soothing profile. The coloured particles may increase flavour perception.

In some examples, the particles comprise a flavourant loaded thereon, such as the first flavourant and/or the second flavourant. In some examples, the flavourant has been pre-loaded onto the particles, i.e. prior to combination of the particles with any of the other components of the pouch content. In some examples, the particles are loaded with the first flavourant. In some examples, the particles are loaded with the second flavourant. In some examples, a first set of particles are loaded with the first flavourant and a second set of particles are loaded with the second flavourant. In this way, the flavour can be associated with the particles and can alter the flavour release profile. For examples, the flavourant being bound to the particles may delay and/or prolong the flavour release, increasing the user's enjoyment over the active lifetime of the article.

In some examples, the particles are formed of a flavoured material. In other words, the particles may be formed of a material which has been mixed with a flavourant, such as the first or second flavourant. In this way, the particles hold the flavourant. In this way, the flavour release and perception may be delayed and/or prolonged.

In some examples, the particles have an average particle size (d50) of between 200 and 2500 µm. In some examples, the particles have an average particle size (d50) of 400 µm or more. In some examples, the particles have an average particle size (d50) of 900 µm or less. In some examples, the particles have a melting point of 40 °C or greater. In some examples, the particles have a melting point of 100 °C or greater. In some examples, the particles have a density, as measured at 25 °C, of 0.4 g/cm³ or greater. In some examples, the particles have a density, as measured at 25 °C, of 0.9 g/cm³ or greater.

In some examples, the particles are contained in an amount by weight, based on the total weight of the content, of between 0.1 wt% and 10 wt%. In some examples, the particles are contained in an amount of between 1 wt% and 3 wt%. In this way, there may be a sufficient quantity of particles in the content to provide a content with beneficial characteristics. When coloured as described herein, the particles may provide a desirable speckled effect which may improve the user's experience.

**Biologically/pharmacologically active compounds** are provided to produce a pharmacological effect in the user. Suitable biologically/pharmacologically active compounds include the group consisting of: nicotine, cocaine, caffeine, opiates and opioids, cathine and cathinone, kavalactones, mysticin, beta-carboline alkaloids, salvinorin A, together with any combinations, functional equivalents to, and/or synthetic alternatives of the foregoing. Biologically/pharmacologically active compounds may also have additive properties.

In some examples the content includes an active compound comprising nicotine and wherein the form of nicotine is selected from the group consisting of nicotine salts, nicotine base, stabilized nicotine and mixtures thereof. For example, the content may include at least one nicotine salt selected from the group consisting of nicotine hydrochloride, nicotine dihydrochloride, nicotine monotartrate, nicotine ditartrate, nicotine ditartrate dihydrate, nicotine sulfate, nicotine zinc chloride monohydrate, nicotine salicylate and mixtures thereof.

**Solvents** may be provided in the OND formulation to improve the mouthfeel of the smokeless article and reduce the amount of saliva wetting required before the user experience begins. The addition of solvents to the precursor blend can also assist with the mixing of the components and ensure that a homogeneous coating of the bulking agent is achieved. Suitable solvents may be any solvent approved for use in food products in the UK and Europe, such as water, saline or water alcohol mixtures. In some cases, the solvent is water.

**Fibrous material** is included in the content of smokeless articles according to the present disclosure. The content comprises at least one fibrous material. In some examples the content may comprise a combination of two or more types of fibrous material. The fibrous material is included as a filler material. Filler materials may be provided to increase the volume of the smokeless article (e.g. by increasing the volume contained within the pouch and to strengthen the contents). In the present proposals, the nature of the filler materials may also impact the release profile of the nicotinic compound and the mouthfeel of the product. Filler materials may also be referred to as "fillers" or "bulking agents". For the avoidance of doubt, the fibrous material is intended to be distinct from the particles described herein.

The fibrous material is composed essentially or completely of fibres, including elongate fibres, fibrous particles, and/or a fibrous powder. The fibrous material may be insoluble in water, so that the fibrous material may comprise or consist of water-insoluble fibres. In some examples, water-insoluble fibres have a water solubility of less than 0.1 gram per 100 mL of water measured at 25 °C and at a pH of 7.0. The fibrous material may be food-grade. The fibrous material may be biocompatible.

The fibrous material may be naturally-occurring (e.g., a plant fibre), bio-derived, or synthetic. In some examples, the fibrous material is naturally-occurring or bio-derived, for example bio-derived. For example, a naturally-occurring fibrous material may be made of collagen, actin, fibrin, starch (e.g., corn starch), cellulose (e.g. powdered cellulose), alginate, or a polyhydroxyalkanoate (PHA), for example cellulose. For example, a bio-derived fibrous powder may be obtained by processing or modifying a natural product. For example, a bio-derived fibrous powder may be made of microcrystalline cellulose (MCC), poly(lactic acid) (PLA), poly(glycolic acid) (PGA), poly(ε-caprolactone), or polybutylene succinate (PBS), for example MCC. The fibrous material may consist of a combination of two or more fibrous materials.

In some examples, the fibrous material is cellulosic. Cellulosic fibres are fibres made of cellulose (e.g. powdered cellulose) or derived from cellulose (e.g. to form MCC). In some examples, the fibrous material comprises or consists of MCC. In some examples, the fibrous material comprises or consists of MCC powder.

In some examples, the fibrous material comprises or consists of fibres. In some examples, the fibrous material comprises or consists of substantially non-spherical fibrous particles or fibres, a fibrous particle being a solid particle made from one or more fibres. In some examples, the fibrous material comprises or consists of substantially spherical fibrous particles or fibres. In some examples, the fibrous material comprises a combination of elongate fibres and fibrous particles (e.g. substantially spherical particles or substantially non-spherical particles), such as a combination of a plant fibre and a fibrous powder. In some examples, the fibrous material is powdered. In some examples, the fibrous material is particulate. In some examples, the fibrous material comprises or consists of particles made of a fibrous material. In some examples, the fibrous material comprises or consists of elongate fibres or fibrous particles. In some examples, the fibrous material comprises or consists of fibrous particles.

The fibrous material may comprise or consist of a plant fibre. A plant fibre is a naturally occurring fibrous material obtained from a plant. It may be chemically unmodified. The plant fibre may be a combination of fibres from different plants. In some examples, the plant fibre is essentially or completely water-insoluble. In some examples, the plant fibre comprises elongate fibres. The plant fibre may be included in the content of the pouch to add strength and structure to the content. Additionally, the plant fibre may function as a natural source of substances such as, for example, biologically/pharmacologically active compounds, flavourants, pH stabilisers etc. The plant fibre may, additionally or alternatively, modify the mouthfeel and/or release profile of the nicotinic compound.

For example, the plant fibre may comprise one or more selected form the group consisting of maize fibres, oat fibres, tomato fibres, barley fibres, rye fibres, sugar beet fibres, buck wheat fibres, wheat fibres, pea fibres, potato fibres, apple fibres, cocoa fibres, bamboo fibres, citrus fibres, pine fibres, eucalyptus fibres, and any combination thereof. In some examples, the plant fibre is selected from bamboo fibres, wheat fibres, or a combination thereof.

Additionally or alternatively, the plant fibre may comprise one or more selected from the group consisting of *Amaranthus dubius, Arctostaphylos uva-ursi* (Bearberry), *Argemone mexicana, Amica, Artemisia vulgaris,* Yellow Tees, *Galea zacatechichi, Canavalia maritima* (Baybean), *Cecropia mexicana* (Guamura), *Cestrum noctumum, Cynoglossum virginianum* (wild comfrey), *Cytisus scoparius, Damiana, Entada rheedii, Eschscholzia califomica* (California Poppy), *Fittonia albivenis, Hippobroma longiflora, Humulus japonica* (Japanese Hops), *Humulus lupulus* (Hops), *Lactuca virosa* (Lettuce Opium), *Laggera alata, Leonotis leonurus, Leonurus cardiaca* (Motherwort), *Leonurus sibiricus* (Honeyweed), *Lobelia cardinalis, Lobelia inflata* (Indian-tobacco), *Lobelia siphilitica, Nepeta cataria* (Catnip), *Nicotiana species* (Tobacco), *Nymphaea alba* (White Lily), *Nymphaea caerulea* (Blue Lily), *Opium poppy, Passiflora incamata* (Passionflower), *Pedicularis densiflora* (Indian Warrior), *Pedicularis groenlandica* (Elephant's Head), *Salvia divinorum, Salvia dorrii* (Tobacco Sage), *Salvia* species (Sage), *Scutellaria galericulata, Scutellaria lateriflora, Scutellaria nana, Scutellaria* species (Skullcap), *Sida acuta* (Wireweed), *Sida rhombifolia, Silene capensis, Syzygium aromaticum* (Clove), *Tagetes lucida* (Mexican Tarragon), *Tarchonanthus camphoratus, Tumera diffusa* (Damiana), *Verbascum* (Mullein), *Zamia latifolia* (Maconha Brava)

In some examples, the plant fibre comprises or consists of bamboo fibre. In some examples, the plant fibre comprises or consists of wheat fibre. In some examples, the fibrous material comprises MCC and one or both of bamboo fibre and wheat fibre.

As used herein, the term "bamboo fibre" refers to natural fibres from plants of the *Bambusoideae* subfamily of the *Poaceae* family of grasses. The fibres may originate from any part of the plant, but in some examples, they originate from the stem of the plant. The fibres may be obtained from commercial sources or may be prepared by grinding or milling plant material until fibres of the required particle size are obtained.

As used herein, the term "wheat fibre" may refer to natural fibres from plants of the *Triticum* genus of the *Poaceae* family of grasses. For example, common wheat, *T. aestivum.* The fibres may originate from any part of the plant, but, in some examples, they originate from the stem of the plant. The fibres may be obtained from commercial sources such as JELUCEL^{®} WF fibres sold by Jelu-werk or VITACEL^{®} WF fibres sold by JRS Pharma. In some examples, the fibres may be prepared by grinding or milling plant material until fibres of the required particle size are obtained.

In some examples, the fibrous material consists of one or more non-tobacco plant fibres. In some examples, the fibrous material comprises a tobacco fibre and one or more other plant fibres.

In some examples, the smokeless article comprises less than 5 wt% tobacco, based on the total weight of the content. In some examples, the smokeless article comprises less than 1 wt% tobacco, based on the total weight of the content. In some examples, the content comprises tobacco in an amount of about 0.1 wt%, about 0.2 wt%, about 0.3 wt%, about 0.4 wt%, or about 0.5 wt%. In some examples, the smokeless article is free or substantially free of tobacco. The presence of tobacco may affect the considerations relating to shelf-life and nicotine absorption.

In some examples, the fibrous material is a nicotine-dosed fibre. As used herein, the term "nicotine-dosed fibre" refers to a composition comprising a fibre and a nicotinic compound. The nicotinic compound may be added to or mixed with the fibre prior to incorporation into the smokeless article. In some examples, the fibres are loaded into a suitable dryer, sprayed with a solution of nicotinic compound and dried to form nicotine-dosed fibres. The dryer may be a fluidised bed dryer. The solution may be a solution of nicotinic compound in water, glycerin, or propylene glycol. In some examples, the solution of nicotinic compound may comprise from 10 to 50 wt% nicotinic compound based on the total solution weight, for example from 10 to 40 wt% or from 10 to 30 wt%.

As used herein, the term "particle size" when referring to the fibrous material indicates the average size of the longest dimension of the fibres or particles, where the average is taken as the median (d50) in a distribution of the longest dimensions of a sample. When the fibrous material comprises generally spherical particles, the longest dimension corresponds to the diameter, and hence the particle size corresponds to the d50 diameter of the sample. For example, a particle size of 50 µm indicates that in the population of fibres, the median (d50) fibre length is 50 µm. Fibres of a desired particle size are available from commercial suppliers such as Jelu-werk. The particle size distribution can be determined using laser diffraction methods (such as described in ISO 13320:2020), by light/electron microscopy static image analysis (such as described in ISO 13322-1:2014), or by sieving methods as described herein.

In some examples, the fibrous material has a monomodal particle size distribution.

The fibres of the fibrous material may have a particle size of 10 to 500 µm, for example 10 to 450 µm, 10 to 400 µm, 10 to 350 µm, 10 to 300 µm, 10 to 250 µm, 10 to 200 µm, 10 to 150 µm, 20 to 150 µm, 30 to 150 µm, 30 to 125 µm, 30 to 100 µm, 30 to 90 µm, 30 to 80 µm, 30 to 75 µm, 30 to 70 µm, 30 to 60 µm, 30 to 50 µm, 30 to 40 µm, 32 to 100 µm, 40 to 150 µm, 40 to 125 µm, 40 to 100 µm, 40 to 90 µm, 40 to 75 µm, 40 to 60 µm, 40 to 50 µm, 10 to 100 µm, 10 to 50 µm, 15 to 50 µm, 10 to 45 µm, 15 to 45 µm, 20 to 50 µm, 20 to 45 µm, 20 to 40 µm, or 25 to 35 µm. In some examples, the fibres have a particle size of about 30 µm. In some examples, the fibres have a particle size of about 40 µm. In some examples, the fibres have a particle size of about 50 µm.

In some examples, the fibres of the fibrous material have a particle size of 15 to 500 µm, for example, 20 to 500 µm, 50 to 500 µm, 50 to 400 µm, 50 to 350 µm, 100 to 350 µm, 150 to 350 µm, 200 to 350 µm, 200 to 300 µm, 225 to 300 µm, 225 to 275 µm, 240 µm to 260 µm, 245 µm to 255 µm, 150 to 250 µm, 160 to 240 µm, 170 to 230 µm, 180 to 220 µm, 190 to 210 µm, 195 to 205 µm, 100 to 500 µm, 150 to 500 µm, 200 to 500 µm, 250 to 500 µm, 250 to 450 µm, 250 to 400 µm, 250 to 350 µm, 260 to 340 µm, 270 to 330 µm, 280 to 320 µm, 290 to 310 µm, or 295 to 305 µm. In some examples, the fibres have a particle size of about 300 µm. In some examples, the fibres have a particle size of about 250 µm. In some examples, the fibres have a particle size of about 200 µm.

In some examples, the fibres of the fibrous material have a particle size of 25 µm or more, 30 µm or more, 32 µm or more, 35 µm or more, 40 µm or more, 45 µm or more, 50 µm or more, 60 µm or more, 70 µm or more, 75 µm or more, 80 µm or more, 90 µm or more, 100 µm or more, 150 µm or more, 175 µm or more, 200 µm or more, 225 µm or more, 250 µm or more, 275 µm or more, 300 µm or more, 325 µm or more, 350 µm or more, 400 µm or more, 500 µm or more, or 600 µm or more. In some examples, the fibres have a particle size of 600 µm or less, 500 µm or less, 450 µm or less, 400 µm or less, 375 µm or less, 350 µm or less, 325 um or less, 300 µm or less, 275 µm or less, 250 µm or less, 225 µm or less, 200 µm or less, 175 µm or less, 150 µm or less, 125 µm or less, 100 µm or less, 90 µm or less, 80 µm or less, 75 µm or less, 70 µm or less, 60 µm or less, 55 µm or less, 50 µm or less, 45 µm or less, 40 µm or less, or 35 µm or less.

In some examples, the fibres of the fibrous material have a particle size of 30 to 300 µm.

In some examples, the fibrous material is contained in an amount by weight, based on the total weight of the content, of between 20 and 80 wt%. In some examples, the amount of the fibrous material is

between 20 and 75 wt%, between 25 and 75 wt%, between 25 and 70 wt%, between 30 and 70 wt%, between 30 and 60 wt%, between 30 and 50 wt%, between 25 and 45 wt%, between 30 and 45 wt%, between 35 and 50 wt%, between 35 and 45 wt%, between 30 and 40 wt%, between 40 and 60 wt%, between 40 and 50 wt%, or between 50 and 60 wt%.

In some examples, the fibrous material is contained in an amount by weight, based on the total weight of the content, of 20 wt% or more, 25 wt% or more, 30 wt% or more, 35 wt% or more, 40 wt% or more, 45 wt% or more, 50 wt% or more, 55 wt% or more, 60 wt% or more, 65 wt% or more, 70 wt% or more, or 75 wt% or more. In some examples, the fibrous material is contained in an amount by weight, based on the total weight of the content, of 80 wt% or less, 75 wt% or less, 70 wt% or less, 65 wt% or less, 60 wt% or less, 55 wt% or less, 50 wt% or less, 45 wt% or less, 40 wt% or less, 35 wt% or less, 30 wt% or less, or 25 wt% or less.

In some examples, the fibres of the fibrous material have a particle size of about 100 µm, 150 µm, 175 µm, 200 µm, 225 µm, 250 µm, 275 µm, 300 µm, 325 µm, 350 µm, 375 µm, or 400 µm. In some examples, the fibres have a particle size of about 200 µm. In some examples, the fibres have a particle size of about 250 µm. In some examples, the fibres have a particle size of about 300 µm.

The content of the pouch may comprise additional fillers. Suitable additional fillers include calcium carbonate, calcium phosphate, corn starch, grains, lactose, polysaccharides (e.g. maltodextrin), polyols, sugars (e.g. dextrose, manitol, sorbitol) and combinations thereof. In some cases, the additional filler content is 5 to 10 wt% of the content e.g. around 6 to 9 wt%.

The content may be formulated to have a predetermined bulk density, when measured at 25 °C. In some examples, the bulk density of the content is 1.0 g/cm³ or less. In some examples, the bulk density is 0.95 g/cm³ or less, 0.9 g/cm³ or less, 0.85 g/cm³ or less, 0.8 g/cm³ or less, 0.75 g/cm³ or less, or 0.7 g/cm³ or less. For example, the bulk density may be 0.8 g/cm³ or less.

**pH stabilisers or adjusters (pH modifiers)** may be provided to adjust the user experience and/or modify the bioavailability of a pharmacologically active compound. For instance, under acidic conditions, nicotine is protonated and does not readily cross mucous membranes. Examples of suitable pH stabilisers include ammonia, ammonium carbonate, sodium carbonate and calcium carbonate. These components may have a buffering action, which helps to maintain the pH at the desired level. In some examples, the overall pH of the smokeless article is pH 7 to pH 9, such as pH 7.25 to pH 8.75 or pH 7.5 to pH 8.5, for example around pH 8.

The pH may be stable for at least 4 months, at least 5 months, at least 6 months, at least 7 months, or at least 8 months. The content (e.g., OND formulation) may have a shelf-life of at least 4 months, at least 5 months, at least 6 months, at least 7 months, or at least 8 months.

The overall pH of a smokeless article may be determined by, for example, (i) placing the smokeless article in 10 mL of distilled water (ii) agitating the mixture for at least 5 minutes and (iii) measuring the pH of the solution with a pH probe.

In some examples, the content comprises a pH modifier (e.g. sodium carbonate) in an amount of from 0 wt% to 1 wt%, for example from 0.01 wt% to 1 wt%, from 0.01 wt% to 0.5 wt%, from 0.01 wt% to 0.2 wt%, from 0.01 wt% to 0.1 wt%, from 0.01 wt% to 0.08 wt%, or from 0.01 wt% to 0.06 wt% based on the total weight of the content.

The pH modifier may have a lower limit of at least 0.01 % by weight of the contents such as at least 0.02 wt%, such as at least 0.03 wt%, such as at least 0.04 wt%, such as at least 0.05 wt%, or such as least 0.06 wt%.

**Salts** may be provided to maintain the osmolarity of the OND formulation within the smokeless article/pouch. Salts contribute to the overall positive user experience of the smokeless article, such as pleasant mouth feel and flavour enhancement. Examples of salts which may be used include sodium chloride, potassium chloride, magnesium chloride and calcium chloride.

**Humectants** may be provided to control moisture content thereby preventing the smokeless article from drying out during storage and reducing the amount of saliva wetting required before the user experience begins. Suitable humectants include polyhydric alcohols (e.g. propylene glycol (PG), triethylene glycol, 1,2-butane diol and glycerol such as vegetable glycerine (VG)) and their esters (e.g. glycerol mono-, di- or tri-acetate). The humectant component may consist of a single humectant compound or may consist of two or more different humectant compounds.

The humectant may have a lower limit of at least 1 % by weight of the contents such as at least 2 wt%, such as at least 5 wt%, such as at least 10 wt%, such as at least 20 wt%, such as at least 30 wt%, or such as least 40 wt%.

The humectant may have an upper limit of at most 70% by weight of the contents, such as at most 65 wt%, such as at most 60 wt%, or such as at most 20 wt%, such as at most 10 wt%, such as at most 5 wt%, such as at most 2 wt%.

In some examples, the amount of humectant is 1 to 70 wt% of the contents, such as 10 to 60 wt% or 50 to 60 wt%.

In some examples, the content has an overall amount of water of between 5 and 60 wt% based on the weight of the contents such as between 40 and 60 wt%, or between 50 and 60 wt%, for example between 52 and 60 wt%.

Smokeless articles having a total moisture content of 10% or less are generally considered to be 'dry'. Smokeless articles having a total moisture content of 40% or more are generally considered to be 'wet'. In some examples, the smokeless article is considered 'wet'. In some examples, the smokeless article is considered 'dry'.

The humectant in the content (e.g. OND formulation) may comprise or consist of water and one or more polyhydric alcohols, for example water and glycerol. In some examples, the humectant may comprise propylene glycol.

**Sweeteners** may be provided to modify the user taste perception and, in particular, overcome bitter flavours that result from other substances. Suitable sweeteners include sucralose, acesulfame K, Stevia, monk fruit, aspartame, maltitol, saccharin sodium, jujube, or a mixture thereof.

In some examples, the content comprises a sweetener (e.g. acesulfame K) in an amount of from 0 wt% to 1 wt%, for example from 0.01 wt% to 1 wt%, from 0.01 wt% to 0.5 wt%, from 0.01 wt% to 0.2 wt%, from 0.01 wt% to 0.1 wt%, from 0.05 wt% to 0.2 wt%, or from 0.05 wt% to 0.15 wt% based on the total weight of the content.

The amount of sweetener may have a lower limit of at least 0.01 % by weight of the content such as at least 0.02 wt%, such as at least 0.03 wt%, such as at least 0.04 wt%, such as at least 0.05 wt%, or such as least 0.06 wt%.

**Stabilisers** are provided to prevent decomposition or degradation over time during storage by, for example, retarding oxidation or unwanted biological activity. Stabilisers may be selected from the group consisting of antioxidants including vitamin E, such as tocopherole, ascorbic acid, sodium pyrosulfite, butylhydroxytoluene, butylated hydroxyanisole, edetic acid and salts thereof; and preservatives including citric acid, tartaric acid, lactic acid, malic acid, acetic acid, benzoic acid, sorbic acid and salts thereof.

**Binders** may be provided. Suitable binders include starches and/or cellulosic binders such as methyl cellulose, ethyl cellulose, hydroxypropyl cellulose, hydroxyethyl cellulose and carboxymethyl cellulose, gums such as xanthan, guar, arabic and/or locust bean gum, organic acids and their salts such as alginic acid (sodium alginate), agar and pectins. In some examples the binder content is 5 to 10 wt% of the contents, e.g. around 6 to 9 wt% or 7 to 8 wt%.

**Colourants** may be provided to modify the user impression of the smokeless article. Colourants may include whitening agents. Colourants may be selected from one or more of common colourants such as curcumin (E100), turmeric (E100(ii)), riboflavin (E101), riboflavin-5'-phosphate (E101(ii)), tartrazine (E102), quinoline yellow (E104), riboflavin-5-sodium phosphate (E106), yellow 2G (E107), sunset yellow FCF (E110), carmine, cochineal (E120), azorubine (E122), amaranth (E123), ponceau 4R (E124), erythrosine (E127), red 2G (E128), allura red AC (E129), patent blue V (E131), indigotine (E132), brilliant blue FCF (E133) chlorophylls (E140), copper complexes of chlorophyll (E141), green S (E142), caramel (E150a-d), brilliant black BN (E151), carbon (E153), brown FK (E154), brown HT (E155), alfa-, beta- and gamma- carotene (E160a), annatto, bixin, norbixin (E160b), bell pepper (Paprika) extract (E160c), lycopene (E160d), beta- apo-8'-carotenal (E160e), ethyl ester of beta-apo-8'-carotenic acid (E160f), flavoxanthin (E161a), lutein (E161b), cryptoxanthin (E161c), rubixanthin (E161d), violaxanthin (E161e), rhodoxanthin (E161f), canthaxanthin (E1619), citranaxanthin (E161h), beetroot extract (E162), anthocyanins (E163), calcium carbonate (E170), titanium dioxide (E171), iron oxides (E172), aluminium (E173), silver (E174), gold (E175), lithol rubine BK (E180), tannins (E181). In some examples, the amount of colourant may be up to about 3% by weight of the smokeless article, such as about 0.5% to about 2.5% or about 1% to about 2%. For the avoidance of doubt, the colourant described in this paragraph is separate to any colourant associated with coloured particles which may be included in some examples.

**Combinations** of any of the features described herein are also envisaged, even when not explicitly described. Included are combinations of features within a single subheading herein, such as combining a specific particle size range and a specific plant fibre within the fibrous material subheading. Also included are combinations of features across multiple subheadings herein, such as combining a specific cooling agent described herein with a specific flavourant described herein, or combining a filler material having a specific size range and/or being a specific material with the presence of one or more of a flavourant, a cooling agent, a colourant, and/or particles. Therefore, any feature described in independent form herein, may be combined any other independent feature insofar as they are compatible and appropriate.

In particular, combinations of properties of the first flavourant and the second flavourant are considered. For example, any one or more of the type, amount, origin and form of the first flavourant may be combined with any specific one or more of the type, amount, origin and form of the second flavourant. Similarly, specific combinations of properties of the cooling agent and the first flavourant are considered.

**In a second aspect,** the present disclosure provides a smokeless article for oral delivery, the smokeless article comprising a pouch enclosing a content. The content comprises a nicotinic compound; at least one fibrous material; a first flavourant comprising eucalyptus flavourant; and a cooling agent distinct from eucalyptol. The first flavourant is present in an amount of between 0.1 and 20 wt% based on the total weight of the content. The cooling agent is contained in an amount of greater than 1 wt% and up to (and including) 10 wt%.

In some examples, the first flavourant is present in an amount of between 0.1 and 15 wt%, for example between 0.1 and 10 wt%, based on the total weight of the content.

The pouch, nicotinic compound, fibrous material and first flavourant are defined according to the first aspect. The cooling agent is defined according to the cooling agent of the first aspect, differing only in that the cooling agent of the second aspect may additionally be or comprise menthol.

In some examples, the cooling agent comprises menthol. In some examples, the cooling agent consists of menthol.

In some examples, the cooling agent comprises one or more selected from: menthol, WS-3, WS-5, WS-23, WS-12, icilin, menthyl lactate, menthoxypropanediol, isopulegol, camphor, borneol, isoborneol, thymol, carvone, and a pentose monosaccharide (e.g. xylitol). In some examples, the cooling agent comprises two or more selected from: menthol, WS-3, WS-5, WS-23, WS-12, icilin, menthyl lactate, menthoxypropanediol, isopulegol, camphor, borneol, isoborneol, thymol, carvone, and a pentose monosaccharide (e.g. xylitol). The pentose monosaccharide may be defined according to the first aspect.

In some examples, the cooling agent comprises menthol and one or more selected from: WS-3, WS-5, WS-23, WS-12, icilin, menthyl lactate, menthoxypropanediol, isopulegol, camphor, borneol, isoborneol, thymol, carvone, and a pentose monosaccharide (e.g. xylitol). In some examples, the cooling agent comprises menthol and WS-5.

In some examples, the first flavourant comprises eucalyptol. In some examples, the first flavourant comprises eucalyptol, and the cooling agent comprises menthol. In some examples, the first flavourant comprises eucalyptol, and the cooling agent comprises menthol and one or more selected from: WS-3, WS-5, WS-23, WS-12, icilin, menthyl lactate, menthoxypropanediol, isopulegol, camphor, borneol, isoborneol, thymol, carvone, and a pentose monosaccharide (e.g. xylitol). In some examples, the first flavourant comprises eucalyptol, and the cooling agent comprises menthol and WS-5.

In some examples, the first flavourant does not comprise eucalyptol.

In some examples, the only component of the content which has a cooling effect is the cooling agent. For example, in some examples, the first flavourant does not have a cooling effect. Similarly, in some examples, such as those comprising a second flavourant, the second flavourant does not have a cooling effect (e.g. does not comprise menthol).

The cooling agent is contained in an amount of greater than 1 wt% and up to (and including) 10 wt%, based on the total weight of the content. In some examples, the amount of the cooling agent is between 1.1 wt% and 10 wt%, between 1.25 wt% and 10 wt%, between 1.5 wt% and 10 wt%, between 2 wt% and 10 wt%, between 2.5 wt% and 10 wt%, between 2.5 wt% and 9 wt%, between 2.5 wt% and 8 wt%, between 2.5 wt% and 7.5 wt%, between 3 wt% and 7.5 wt%, between 3 wt% and 7 wt%, between 4 wt% and 7 wt%, between 4 wt% and 6 wt%, between 4 wt% and 5 wt%, between 5 wt% and 6 wt%, or between 4.5 wt% and 6.5 wt%. In some examples, the amount is between 2 wt% and 8 wt%. In some examples, the amount is between 2.5 wt% and 7.5 wt%. In some examples, the amount is between 3 wt% and 7 wt%. In some examples, the amount is between 4 wt% and 6 wt%.

In some examples, the weight ratio of the cooling agent to the first flavourant ([weight cooling agent] / [weight first flavourant]) is between 0.1 and 10. In some examples, the weight ratio is between 0.1 and 9, between 0.1 and 8, between 0.1 and 7.5, between 0.1 and 6, between 0.1 and 5, between 0.25 and 5, between 0.3 and 5, between 0.4 and 5, between 0.5 and 5, between 0.5 and 4, between 0.5 and 3, between 0.5 and 2.5, between 0.5 and 2, between 0.5 and 1.75, between 0.5 and 1.5, between 0.5 and 1.25, between 0.75 and 1.5, or between 0.75 and 1.25. In some examples, the weight ratio is between 0.25 and 2.5. In some examples, the weight ratio is between 0.25 and 2. In some examples, the weight ratio is between 0.25 and 1.5. In some examples, the weight ratio is between 0.5 and 1.5. In some examples, the weight ratio is between 0.5 and 1. In some examples, the weight ratio is between 1 and 1.5. In some examples, the weight ratio is between 0.75 and 1.25.

It has been found that, when a pouch content comprises a combination of a first flavourant comprising eucalyptus flavourant and a cooling agent distinct from eucalyptol in an amount of greater than 1 wt%, the smokeless article can provide improved organoleptic properties for a favourable and improved user experience. For example, such a product may result in a particularly "fresh" and "cooling" product. Additionally or alternatively, such a combination of a eucalyptus flavourant and a cooling agent may result in a particularly soothing product and/or a particularly favourable sweetness profile during use (i.e., a temporal soothing profile or sweetness profile). These properties are particularly prominent for the combination of eucalyptus flavourant and menthol or a cooling agent with similar or stronger cooling properties than menthol. It has been found that such properties are particularly prevalent, or even enhanced, when a second non-eucalyptol cooling agent is also included. That is, an oral product having a combination of a eucalyptus flavourant, a first cooling agent (such as menthol), and a second cooling agent other than eucalyptol or menthol may provide a particularly "fresh" and "cooling" user experience, with a unique time-dependent flavour and/or aroma distribution as well as providing soothing to the user's mouth.

The contents according to the second aspect may further be as defined according to the first aspect. For example, they may comprise one or more additional flavourants (e.g., second and third flavourant(s)), biologically/pharmacologically active compounds, solvents, particles, fibrous material, fillers, salts, humectants, sweeteners, stabilisers, pH stabilisers or adjusters (pH modifiers), binders, and/or colourants as defined according to the first aspect or may otherwise have features (e.g., amounts, ratios) as defined for the first aspect.

**In a third aspect,** the present disclosure provides a smokeless article for oral delivery, the smokeless article comprising a pouch enclosing a content. The content comprises a nicotinic compound; at least one fibrous material; a first flavourant comprising eucalyptus flavourant; and a second flavourant, wherein the ratio of the weight of the second flavourant to the weight of the first flavourant is between 0.01 and 1.

The pouch, nicotinic compound, fibrous material, the first flavourant and the second flavourant are defined according to the first aspect.

It has been found that, when the content comprises a first flavourant comprising eucalyptus flavourant, and a second flavourant, wherein the ratio of the weight of the second flavourant to the weight of the first flavourant is between 0.01 and 1, the desired properties of each flavourant can simultaneously be exhibited in balance, without compromising soothing and cooling sensations from the first flavourant comprising eucalyptus flavourant. To elaborate, when the ratio of the weight of the second flavourant to the weight of the first flavourant is between 0.01 and 1, a particularly favourable and unique flavour profile can be exhibited without diminishing any soothing or cooling sensations from the first flavourant comprising eucalyptus flavourant. When the ratio is less than 0.01, the contribution of the second flavourant on the overall flavour profile may be imperceptible to a user (when using a smokeless article according to the third aspect). Additionally, when the ratio is less than 0.01, a user may expect the second flavourant to be imperceptible compared to the first flavourant, causing a user not to experience any or all properties mental associated with the second flavourant, as part of the overall characteristics of the content. When the ratio is above 1, soothing and cooling sensations from the first flavourant may be unfavourably diminished or nullified, resulting in a less favourable balance of properties and an overall less favourable user experience.

In some examples, the first flavourant consists of eucalyptus flavourant.

In some examples, the ratio of the weight of the second flavourant to the weight of the first flavourant is defined in the same way as the first ratio defined herein. In some examples, the ratio of the weight of the second flavourant to the weight of the first flavourant is between 0.01 and 0.5, for example between 0.01 and 0.2, for example between 0.1 and 0.2. In some cases, the ratio is between 0.1 and 0.2. In this way, a particularly favourable balance of flavours and properties (e.g., soothing properties) can be exhibited, for improving a user experience.

In some examples, the content further comprises a third flavourant. The third flavourant may be as defined above for the first aspect. For example, when the content comprises a third flavourant, each of the first flavourant, the second flavourant, and the third flavourant may present in an amount as defined above, for example between 0.1 wt% and 10 wt% based on the total weight of the content.

Additionally, when the content comprises a third flavourant, a ratio may be used to define each of an amount of the second flavourant to the first flavourant, and an amount of the third flavourant to the first flavourant. Suitable ratios for the amount of the third flavourant to the first flavourant may be as defined for the first aspect (e.g. as defined for the first ratio defined herein).

The contents according to the third aspect may further be as defined according to the first aspect. For example, they may comprise one or more further flavourants, a cooling agent, pentose monosaccharide, biologically/pharmacologically active compounds, solvents, particles, fibrous material, fillers, salts, humectants, sweeteners, stabilisers, pH stabilisers or adjusters (pH modifiers), binders, and/or colourants as defined according to the first aspect or may otherwise have features as defined for the first aspect, such as the types and amounts of each flavourant and/or the total amount of flavourant.

**In a fourth aspect,** the present disclosure provides a smokeless article for oral delivery, the smokeless article comprising a pouch enclosing a content. The content comprises a nicotinic compound; at least one fibrous material; a first flavourant selected from eucalyptus oil, eucalyptus extract, eucalyptus butter, eucalyptus powder, eucalyptus gel, and eucalyptus concentrate; and one or more eucalyptus compounds selected from the group consisting of 1,8-cineole, macrocarpal-C, p-cymene, α-pinene, β-pinene, limonene, citronellal, citral, eudesmol, terpinen-4-ol, terpineol, α-phellanderene, and 9β-sitosterol.

The pouch, nicotinic compound and fibrous material are defined according to the first aspect.

By providing a content comprising a first flavourant comprising 'natural' eucalyptus (e.g., eucalyptus oil, eucalyptus extract, eucalyptus butter, eucalyptus powder, eucalyptus gel, and eucalyptus concentrate) in combination with one or more eucalyptus compounds (which may also be considered natural eucalyptus, but refers specifically to one or more isolated compounds naturally found in *Eucalyptus*) selected from the group consisting of 1,8-cineole, macrocarpal-C, p-cymene, α-pinene, β-pinene, limonene, citronellal, citral, eudesmol, terpinen-4-ol, terpineol, α-phellanderene, and 9β-sitosterol (as supplementary eucalyptus compounds), the content may exhibit a particularly favourable balance of flavour and soothing effects. As explained above, it is believed that these eucalyptus compounds may contribute to the soothing and/or cooling sensations for providing a particularly favourable user experience. By providing (supplemental) eucalyptus compounds, a balance of soothing and cooling effects may be enhanced compared to contents comprising a first flavourant only (wherein the first flavourant is a natural eucalyptus flavourant, such as eucalyptus oil). This is because contents comprising a natural eucalyptus flavourant and supplemental eucalyptus compounds comprise said eucalyptus compounds in elevated ratios compared to contents that do not comprise supplemental eucalyptus compounds.

In some examples, the first flavourant is eucalyptus oil or eucalyptus extract.

In some examples, the one or more eucalyptus compounds may be selected from the group consisting of 1,8-cineole, macrocarpal-C, p-cymene, α-pinene, limonene, and terpinen-4-ol.

In some examples, the one or more eucalyptus compounds comprises 1,8-cineole. In some examples, the one or more eucalyptus compounds comprises macrocarpal-C. As explained previously herein, Macrocarpal C is not typically found in certain species of *Eucalyptus,* including *E*. *globulus* (which is a common source of eucalyptus oil), therefore it may be desired to add a supplemental amount of macrocarpal C to impart its desirable properties. In some examples, the one or more eucalyptus compounds comprises α-pinene. In some examples, the one or more eucalyptus compounds comprises p-cymene. In some examples, the one or more eucalyptus compounds comprises limonene. In some examples, the one or more eucalyptus compounds comprises terpine-4-ol.

In some examples, the one or more eucalyptus compounds are present in an amount of between 0.1 wt% and 5 wt% based on the total weight of the content. An amount of each of the one or more eucalyptus compounds may be independently selected. In this way, the balance of properties, for example a flavour profile and soothing and cooling properties, exhibited by the content may be finely tuned for offering a particularly pleasant user experience. For example, when the content comprises one or more eucalyptus compounds selected from the group consisting of 1,8-cineole, macrocarpal-C, p-cymene, α-pinene, limonene, and terpinen-4-ol, each of the one or more eucalyptus compounds may be present in an amount of between 0.1 wt% and 2 wt%. In some examples, each of the one or more eucalyptus compounds may be present in an amount of between 0.1 wt% and 1 wt%, for example between 0.1 wt% and 0.5 wt%.

The contents according to the fourth aspect may further be as defined according to the first aspect. For example, they may comprise one or more flavourants, cooling agent, pentose monosaccharides, biologically/pharmacologically active compounds, solvents, particles, fibrous material, fillers, salts, humectants, sweeteners, stabilisers, pH stabilisers or adjusters (pH modifiers), binders, and/or colourants as defined according to the first aspect, or may otherwise have features as defined for the first aspect.

**In a fifth aspect,** the present disclosure provides a smokeless article for oral delivery, the smokeless article comprising a pouch enclosing a content. The content comprises a nicotinic compound; at least one fibrous material; a first flavourant comprising synthetic eucalyptus flavourant; and a second flavourant comprising natural eucalyptus.

The pouch, nicotinic compound, fibrous material, synthetic eucalyptus and natural eucalyptus are defined according to the first aspect.

Natural eucalyptus and synthetic eucalyptus are as defined herein with respect to the first aspect. For example, the natural eucalyptus may comprise one or more eucalyptus compounds selected from the group consisting of 1,8-cineole, macrocarpal-C, p-cymene, α-pinene, β-pinene, limonene, citronellal, citral, eudesmol, terpinen-4-ol, terpineol, α-phellanderene, and 9β-sitosterol.

By providing a content comprising a first flavourant comprising synthetic eucalyptus flavourant, and one or more eucalyptus extract compounds selected from the group consisting of 1,8-cineole, macrocarpal-C, p-cymene, α-pinene, β-pinene, limonene, citronellal, citral, eudesmol, terpinen-4-ol, terpineol, α-phellanderene, and 9β-sitosterol, the content (and in turn the smokeless article) can emulate a content comprising natural eucalyptus flavourant (e.g., eucalyptus oil), without using a first flavourant comprising natural eucalyptus (e.g., eucalyptus extract). This has the benefit of emulating natural eucalyptus flavourants, without providing eucalyptus compounds that could diminish any soothing or cooling sensations, or which could otherwise lead to a less favourable flavour profile. That is, by supplementing synthetic eucalyptus with one or more of the above eucalyptus compounds to emulate natural eucalyptus flavourants, the desirable characteristics of a natural eucalyptus flavourant (e.g., soothing and freshness) may be enhanced due to the absence of eucalyptus compounds which could otherwise lead to a less favourable user experience.

In some examples, the first flavourant consists of synthetic eucalyptus flavourant. In some examples, the first flavourant consists of artificial eucalyptus flavourant.

In some examples, the one or more eucalyptus compounds may be selected from the group consisting of 1,8-cineole, macrocarpal-C, p-cymene, α-pinene, limonene, and terpinen-4-ol. In some examples, the one or more eucalyptus compounds comprises 1,8-cineole. In some examples, the one or more eucalyptus compounds comprises macrocarpal-C. As explained previously herein, Macrocarpal C is not typically found in certain species of *Eucalyptus,* including *E. globulus* (which is a common source of eucalyptus oil), therefore it may be desired to add a supplemental amount of macrocarpal C to impart its desirable properties. In some examples, the one or more eucalyptus compounds comprises α-pinene. In some examples, the one or more eucalyptus compounds comprises p-cymene. In some examples, the one or more eucalyptus compounds comprises limonene. In some examples, the one or more eucalyptus compounds comprises terpine-4-ol.

In some examples, the one or more eucalyptus compounds are present in an amount of between 0.1 wt% and 5 wt% based on the total weight of the content. An amount of each of the one or more eucalyptus compounds may be independently selected. In this way, the balance of properties, for example a flavour profile and soothing and cooling properties, exhibited by the content may be finely tuned for offering a particularly pleasant user experience. For example, when the content comprises one or more eucalyptus compounds selected from the group consisting of 1,8-cineole, macrocarpal-C, p-cymene, α-pinene, limonene, and terpinen-4-ol, each of the one or more eucalyptus compounds may be present in an amount of between 0.1 wt% and 2 wt% based on the total weight of the content. In some examples, each of the one or more eucalyptus compounds may be present in an amount of between 0.1 wt% and 1 wt%, for example between 0.1 wt% and 0.5 wt%. By way of example, when the content comprises 1,8-cineole, macrocarpal C, and limonene, 1,8-cineole may be present in an amount of between 0.1 wt% and 0.5 wt%, the macrocarpal C may be present in an amount of between 0.1 wt% and 2 wt%, and the limonene may be present in an amount of between 0.1 wt% and 1 wt% based on the total weight of the content.

The contents according to the fifth aspect may further be as defined according to the first aspect. For example, they may comprise one or more flavourants, cooling agent, pentose monosaccharides, biologically/pharmacologically active compounds, solvents, particles, fibrous material, fillers, salts, humectants, sweeteners, stabilisers, pH stabilisers or adjusters (pH modifiers), binders, and/or colourants as defined according to the first aspect, or may otherwise have features as defined for the first aspect.

**In a sixth aspect,** the present disclosure provides a smokeless article for oral delivery, the smokeless article comprising a pouch enclosing a content. The content comprises a nicotinic compound in an amount of between 0.1 wt% and 5 wt%; at least one fibrous material in an amount of between 20 wt% and 80 wt%; a first flavourant comprising eucalyptus flavourant in an amount of between 0.1 wt% and 20 wt%; and water in an amount of between 1 wt% and 65 wt%, wherein the amount of nicotinic compound, at least one fibrous material, first flavourant, and water are based on a total weight of the content.

The pouch, nicotinic compound, fibrous material, and eucalyptus flavourant are defined according to the first aspect.

In some examples, the nicotinic compound may be present in an amount of between from 0.3 wt% to 2 wt%, for example from 0.4 wt% to 2 wt%, from 0.5 wt% to 2 wt%, from 0.5 wt% to 1.9 wt%, from 0.8 wt% to 1.8 wt%, from 1 wt% to 1.6 wt%, from 1.2 wt% to 1.6 wt%, or from 1.3 wt% to 1.5 wt%, based on the total weight of the content.

In some examples, the at least one fibrous material may be present in an amount of between 20 wt% and 60 wt%, for example between 30 wt% and 60 wt%, for example between 40 wt% and 60 wt%, for example between 40 wt% and 50 wt%.

In some examples, the first flavourant may be present in an amount of between 0.1 wt% and 10 wt%, for example between 0.1 wt% and 8 wt%, for example between 0.1 wt% and 6 wt%, for example between 0.1 wt% and 5 wt%, for example between 0.5 wt% and 5 wt%, for example between 0.5 wt% and 4 wt%, for example between 0.5 wt% and 3 wt%, for example 0.5 wt% and 2 wt%, for example between 0.5 wt% and 1.5 wt%, or for example between 1.0 wt% and 1.5 wt%.

In some examples, water may be present in an amount of between from 30 wt% to 60 wt%, for example from 35 wt% to 60 wt%, from 40 wt% to 60 wt%, from 40 wt% to 55 wt%, from 40 wt% to 50 wt%, or from 42 wt% to 50 wt% based on the total weight of the content. In some examples, the amount of water in the content is from 42 wt% to 48 wt%, based on the total weight of the content.

In some examples, the nicotinic compound is one selected from the group consisting of nicotine, a nicotine salt, a nicotine complex, and a nicotine solvate.

In some examples, the at least one fibrous material may comprise collagen, actin, fibrin, starch (e.g., corn starch), cellulose (e.g. powdered cellulose), alginate, or a polyhydroxyalkanoate (PHA), for example cellulose. For example, a bio-derived fibrous powder may be obtained by processing or modifying a natural product. For example, a bio-derived fibrous powder may be made of microcrystalline cellulose (MCC), poly(lactic acid) (PLA), poly(glycolic acid) (PGA), poly(ε-caprolactone), or polybutylene succinate (PBS), for example MCC. The fibrous material may consist of a combination of two or more fibrous materials. In some examples, the one or more fibrous materials is MCC.

In some examples, the content further comprises menthol in an amount of between 0.5 wt% and 10 wt%. In some examples, the amount of menthol is from 0.75 wt% to 10 wt%, from 1 wt% to 10 wt%, from 1 wt% to 9 wt%, from 1 wt% to 7.5 wt%, from 2 wt% to 7.5 wt%, from 2.5 wt% to 7.5 wt%, from 3 wt% to 7 wt%, from 4wt% to 7 wt%, from 5 wt% to 7 wt%, from 4 wt% to 6 wt%, from 4 wt% to 5wt%, or from 5 wt% to 6 wt%.

In some examples, the content further comprises a cooling agent distinct from eucalyptol or menthol (e.g. as described with respect to the first aspect) in an amount of between 0.05 wt% and 2 wt%. In some examples, the amount of cooling agent is from 0.075 wt% to 1.5 wt%, from 0.1 wt% to 1.5 wt%, from 0.1 wt% to 1.25 wt%, from 0.1 wt% to 1 wt%, from 0.15 wt% to 1 wt%, from 0.2 wt% to 1 wt%, from 0.2 wt% to 0.8 wt%, from 0.2 wt% to 0.7 wt%, from 0.2 wt% to 0.6 wt%, from 0.25 wt% to 0.6 wt%, from 0.3 wt% to 0.6 wt%, or from 0.3 wt% to 0.5 wt%.

The content according to the sixth aspect may further be as defined according to the first aspect. For example, the content may further comprise a second flavourant. For example, the content may comprise one or more flavourants, cooling agents, pentose monosaccharide, biologically/pharmacologically active compounds, solvents, particles, fibrous material, fillers, salts, humectants, sweeteners, stabilisers, pH stabilisers or adjusters (pH modifiers), binders, and/or colourants as defined according to the first aspect, or may otherwise have features as defined for the first aspect. For example, the content may further comprise a second flavourant and optionally a third flavourant as defined for the first aspect.

**In a seventh aspect,** the present disclosure provides a method of manufacturing a smokeless article for oral consumption, the smokeless article comprising a pouch enclosing a content.

In some examples, the method comprises the steps: (a) mixing a nicotinic compound, at least one fibrous material, a first flavourant comprising eucalyptus flavourant, a second flavourant comprising a non-eucalyptus flavourant, and a cooling agent distinct from menthol and eucalyptol, to form a content; (b) adding the content to the pouch or forming the pouch around the content. After adding the content to the pouch or forming the pouch around the content, the method may further comprise a step (c) of thermally or chemically sealing the pouch material to enclose said contents.

In some examples, the method may therefore comprise (a) providing a content according to the first aspect, and (b) adding the content to a pouch or forming a pouch around the content.

In some examples, the smokeless article produced by the method is a smokeless article according to the first aspect. Accordingly, in step (a), in some examples, the first flavourant may be added up to an amount of 0.1 and 10 wt.% based on the total weight of the content, and the second flavourant may be added up to an amount of between 0.1 and 10 wt.% based on the total weight of the content. Furthermore, in step (a), in some examples, the first flavourant may be added up to an amount specified for the first and second flavourants of the first aspect.

In some examples, the first flavourant and the second flavourant are mixed together before step (a) to form a flavourant premix. The flavourant premix may then be added to the mixture during step (a). In some examples, the cooling agent is subsequently added to the flavourant premix before step (a). In this way, both flavourants may be distributed evenly during step (a). In some examples, the cooling agent is added to the mixture during step (a) (i.e., it is not added to the flavourant premix prior to step (a)).

Each of the first flavourant and second flavourant may independently be provided as a solid, a liquid, a dispersion, or a solution. In some examples, the first flavourant is a liquid (e.g. when the first flavourant is eucalyptus oil or artificial eucalyptus). In some examples, the second flavourant is a solid (e.g. when the second flavourant is menthol). In some examples, the first flavourant is a liquid and the second flavourant is a solid. In some examples, the first flavourant is a liquid and the second flavourant is a solid, and the first flavourant and the second flavourant are mixed together before step (a) to form a flavourant premix.

In some examples, step (a) further comprises adding a third flavourant to form the content. The third flavourant may be any flavourant described herein. When step (a) further comprises adding a third flavourant to form the content, each of the first flavourant, the second flavourant, and the third flavourant may be added in an amount of between 0.1 and 10 wt.% based on the total weight of the content.

In some examples, in step (a), the nicotinic compound and the at least one fibrous material are mixed prior to the addition of flavourants. In this way, the flowability or mixability of the content may be improved, in particular if one or more of the flavourants are in a liquid form or are in a solid form having a high water content.

In some examples, the fibrous material is dosed with the nicotinic compound, for example as a nicotinic solution, prior to the addition of the flavourants.

In some examples, the components of the content are mixed using a blender (e.g. a food processor or similar for small scale production). In some examples, the content resulting from step (a) is isotropic. That is, the components are evenly dispersed (e.g. they may appear to be homogeneously mixed on the macroscopic scale).

In some examples, the method further comprises adding particles (e.g. coloured particles) to the content. In some examples, the particles are mixed with the at least one fibrous material prior to step (a). In some examples, the particles are mixed with the at least one fibrous material during step (a). In some examples, the particles are added to the content after step (a). In some examples, the method further comprises a step of loading a plurality of particles with the first flavourant and/or the second flavourant prior to step (a). In some examples, the method further comprises a step of loading a first plurality of particles with the first flavourant and loading a second plurality of particles with the second flavourant prior to step (a), and then adding the first and second plurality of particles to the content.

In some examples, when the content comprises a plurality of particles, the method may further comprise a step of loading the plurality of particles with the first flavourant and/or the second flavourant. In such examples, the particles loaded with flavourant(s) may be subsequently mixed with flavourants and/or other components. In some examples, the particles are formed from a flavoured material (i.e. a material which has been combined with a flavourant) to form flavoured particles directly. For example, the material of the particles may be mixed with a flavourant, and the mixture then extruded to form flavourant particles.

In some examples, when the content comprises a plurality of particles, the process further comprises a step of modifying the colour of the content by providing coloured particles. In some examples, uncoloured particles are mixed with a colourant to form coloured particles. In some examples, coloured particles having a first colour are mixed with a colourant to provide coloured particles having a second, different colour to the first colour. In some examples, the particles are formed from a coloured material (such as a material which has already been combined with a colourant, or a material which is intrinsically coloured) to form coloured particles directly. For example, the material of the particles may be mixed with a colourant, and the mixture then extruded to form coloured particles.

In some examples, the method may further comprise a step of adding a pentose monosaccharide. In some examples, step (a) further comprises adding a pentose monosaccharide to form the content, such as xylitol.

Optionally, the method may include adding further additives to the content. The additives may include, but are not limited to, one or more selected from: additional fillers, pH modifiers, humectants, sweeteners, stabilisers, binders, and colourants. Further additives may be added before, during, or after step (a).

**In an eighth aspect,** the present disclosure provides a use of a first flavourant and a second flavourant in a content of a smokeless article for oral consumption, the first flavourant comprising eucalyptus flavourant, to improve the user experience. The first flavourant and second flavourant may be according to the flavourants of the first aspect, respectively, including in amount (wt%) of and/or ratio of first and second flavourants. The use may further include using a cooling agent (e.g. as defined in the first aspect) to improve the user experience (such as user perception of freshness).

**In a ninth aspect,** the present disclosure provides a kit comprising a plurality of smokeless articles according to the first aspect, the second aspect, the third aspect, the fourth aspect, the fifth aspect, or the sixth aspect, or a plurality of smokeless articles produced according to the seventh aspect.

In some examples, the kit further comprises a container, wherein the container removably encloses said plurality of smokeless articles.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

The preceding summary is provided for purposes of summarizing some examples to provide a basic understanding of aspects of the subject matter described herein. Accordingly, the above-described features should not be construed to narrow the scope or spirit of the subject matter described herein in any way. Moreover, the above and/or proceeding examples may be combined in any suitable combination to provide further examples, except where such a combination is clearly impermissible or expressly avoided. Other features, aspects, and advantages of the subject matter described herein will become apparent from the following text and the accompanying drawings.

### BRIEF DESCRIPTION OF THE FIGURES

Aspects, features and advantages of the present disclosure will become apparent from the following description of examples in reference to the appended drawings in which like numerals denote like elements.
**Figure 1** shows a cross sectional view of a first embodiment of a smokeless article.
**Figure 2** shows a cross sectional view of a second embodiment of a smokeless article.
**Figure 3** shows a cross sectional view of a third embodiment of a smokeless article.

### DETAILED DESCRIPTION OF EMBODIMENTS

Before describing several examples implementing the present disclosure, it is to be understood that the present disclosure is not limited by specific construction details or process steps set forth in the following description and accompanying drawings. Rather, it will be apparent to those skilled in the art having the benefit of the present disclosure that the systems, apparatuses and/or methods described herein could be embodied differently and/or be practiced or carried out in various alternative ways.

Unless otherwise defined herein, scientific and technical terms used in connection with the presently disclosed inventive concept(s) shall have the meanings that are commonly understood by those of ordinary skill in the art, and known techniques and procedures may be performed according to conventional methods well known in the art and as described in various general and more specific references that may be cited and discussed in the present specification.

Any patents, published patent applications, and non-patent publications mentioned in the specification are hereby incorporated by reference in their entirety.

All examples implementing the present disclosure can be made and executed without undue experimentation in light of the present disclosure. While particular examples have been described, it will be apparent to those of skill in the art that variations may be applied to the systems, apparatus, and/or methods and in the steps or in the sequence of steps of the methods described herein without departing from the concept, spirit, and scope of the inventive concept(s). All such similar substitutions and modifications apparent to those skilled in the art are deemed to be within the spirit, scope, and concept of the inventive concept(s) as defined by the appended claims.

The use of the term "a" or "an" in the claims and/or the specification may mean "one," as well as "one or more," "at least one," and "one or more than one." As such, the terms "a," "an," and "the," as well as all singular terms, include plural referents unless the context clearly indicates otherwise. Likewise, plural terms shall include the singular unless otherwise required by context.

The use of the term "or" in the present disclosure (including the claims) is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used in this specification and claim(s), the words "comprising, "having," "including," or "containing" (and any forms thereof, such as "comprise" and "comprises," "have" and "has," "includes" and "include," or "contains" and "contain," respectively) are inclusive or open-ended and do not exclude additional, unrecited elements or method steps.

Unless otherwise explicitly stated as incompatible, or the physics or otherwise of the embodiments, examples, or claims prevent such a combination, the features of examples disclosed herein, and of the claims, may be integrated together in any suitable arrangement, especially ones where there is a beneficial effect in doing so. This is not limited to only any specified benefit, and instead may arise from an "ex post facto" benefit. This is to say that the combination of features is not limited by the described forms, particularly the form (e.g. numbering) of example(s), embodiment(s), or dependency of claim(s). Moreover, this also applies to the phrase "in one embodiment," "according to an embodiment," and the like, which are merely a stylistic form of wording and are not to be construed as limiting the following features to a separate embodiment to all other instances of the same or similar wording. This is to say, a reference to 'an,' 'one,' or 'some' embodiment(s) may be a reference to any one or more, and/or all embodiments, or combination(s) thereof, disclosed. Also, similarly, the reference to "the" embodiment may not be limited to the immediately preceding embodiment. Further, all references to one or more embodiments or examples are to be construed as non-limiting to the claims.

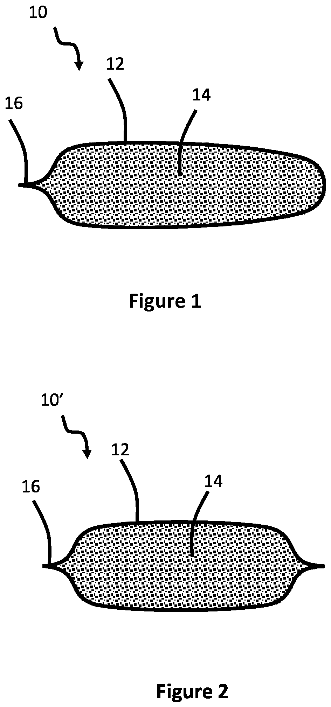

As shown in Figure 1 there is provided a first embodiment of a smokeless article 10 having a pouch 12 containing contents 14. The pouch 12 is substantially rectangular. The pouch 12 is formed from a single sheet of material and is substantially filled by the contents 14. The pouch 12 has a seal 16 along each of the three edges where the inner face of the single sheet meets itself to seal the contents 14 in the pouch 12.

Figure 2 shows a second embodiment of a smokeless article 10' having a pouch 12 containing contents 14. The pouch 12 is substantially circular. The pouch 12 is formed from two opposing sheets of material and is substantially filled by the contents 14. The pouch has a circumferential seal 16 along the edges where the two opposing sheets of material meet to seal the contents 14 in the pouch 12.

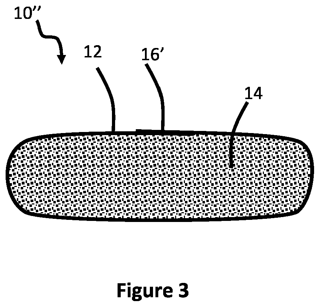

Figure 3 shows a third embodiment of a smokeless article 10" that, like the first embodiment, has a pouch 12 made from a single sheet of material. However, one of the three seals 16' is formed by an overlap of the inner face and the outer face of the single sheet meet to seal the contents 14 in the pouch 12. The remaining two seals at opposing ends of the pouch 12 are formed where the inner face of the single sheet meets itself.

### Preparation of formulations

The example formulations were prepared according to the following method.
1. All the materials were dispensed into appropriately sized beakers using a 2 d.p. balance to determine the weights.
2. Fibrous material was dry mixed in a mixer.
3. Cold water was added to the mixer for wet mixing.
4. A nicotine solution was added to the mixer, and mixed.
5. A soda solution was added to the mixer, and mixed.
6. An ammonium chloride solution was added to the mixer, and mixed.
7. The first and second flavourant were mixed together to form a flavourant premix, a cooling agent (different from eucalyptol and menthol) was then added to the flavourant premix, and the flavourant premix was added to the mixer and mixed.
8. The blend was removed from the mixer.

After preparation of a formulation, any additional components (if necessary) (e.g., particles) can be mixed with the formulation to form a content. Once a formulation comprises all required components it may correspond to a content and may then be enclosed by a pouch or added to a pouch, and subsequently sealed (e.g., by sealing).

### Example formulations

The example formulations contained the following components.

### Formulation 1

| **Material** | **%w/w** |
|---|---|
| Nicotine | 0.5 to 1.0 |
| Solvent (for nicotine and/or other components; e.g., water) | 35 to 40 |
| Bulking agent(s) (e.g., MCC) | 35 to 57 |
| Humectant(s) | 4 to 13 |
| pH regulator (E.g., soda / sodium carbonate) | 0.1 to 0.6 |
| Artificial eucalyptus flavourant | 2 to 6 |
| Second flavourant (e.g. menthol) | 2 to 6 |
| Cooling agent (e.g. WS-5) | 0.2 to 0.6 |
| Xylitol | 0 to 5 |
| **Total** | **100.00** |

In a first test where a set of users were asked to rate properties of smokeless articles based on article information (for example, flavour details), users reported a stronger positive mental association between soothing properties and articles which were described as comprising "eucalyptus" flavourants, compared to other flavourants. In particular, users reported a stronger positive mental association between gum soothing properties and articles which were described as comprising eucalyptus flavourants. Users also reported a mental association with cooling ('fresh') effects and articles which were described as comprising mint flavourant(s), such as peppermint.

In a second test, a set of users were each given a first smokeless article comprising a pouch filled with a formulation (content) according to table 1 (comprising artificial eucalyptus flavourant, menthol, and WS-5). Users were also given a second smokeless article comprising a pouch filled with a content analogous to the first smokeless article, differing only in eucalyptus oil being used as the first flavourant instead of artificial eucalyptus flavour. The users were also given other smokeless articles which did not comprise a eucalyptus flavourant (e.g. only comprising a mint flavourant, such as peppermint). In user preference tests, the two samples containing a eucalyptus flavourant were preferred with respect to overall liking, taste liking, refreshing effect in mouth, and aftertaste, due to a particularly favourable flavour profile and soothing, cooling, and moisturising properties. Users reported a particular preference for articles comprising the artificial eucalyptus flavourant compared to those with eucalyptus oil, and especially to those which did not comprise eucalyptus flavourant, also due to a particularly favourable flavour profile and soothing, cooling, and moisturising properties. Some smokeless articles further comprised xylitol, while others did not comprise xylitol. Users reported a strong preference for articles comprising xylitol as compared to articles which did not comprise xylitol, due to a favourable flavour profile, and soothing or cooling sensations. Some smokeless articles further comprised coloured particles, while others did not comprise coloured particles. Users reported a strong preference for articles comprising coloured particles as compared to articles which did not comprise coloured particles, due to a perception of clean, cool, and fresh mouthfeel, which lasts longer compared variants without particles.

In a third test, a set of users were each given a first smokeless article comprising a pouch filled with a formulation (content) according to table 1 (comprising artificial eucalyptus flavourant, menthol, and WS-5). Users were also given a second smokeless article comprising a pouch differing from the first smokeless article in that it comprised a lower amount of the second flavourant (menthol) of 0.5 to 1 wt%, and it contained no additional cooling agent. Users reported that the first article provided an immediate and high intensity cooling effect with a strong feeling of mouth freshness, whereas the second article provided a less significant cooling effect and a less significant feeling of mouth freshness. Additionally, in the second smokeless article, the eucalyptus flavourant contained an amount of liquorice flavourant mixed therewith, whereas the eucalyptus flavourant of the first article did not. It was found that the first article possessed a very strong eucalyptus flavour, whereas the eucalyptus flavour of the second article was almost imperceivable, meaning that the mental association between eucalyptus and freshness/cooling was lacking in the second article. Users reported that the second article possessed a salty and liquorice-like flavour.

## Claims

1. A smokeless article for oral consumption, the smokeless article comprising a pouch enclosing a content, the content comprising:
a nicotinic compound;
at least one fibrous material;
a first flavourant comprising eucalyptus flavourant;
a second flavourant comprising a non-eucalyptus flavour component; and
a cooling agent distinct from menthol and eucalyptol; and
wherein:
the first flavourant is present in an amount of between 0.1 and 20 wt% based on the total weight of the content, the second flavourant is present in an amount of between 0.1 and 20 wt% based on the total weight of the content, and the cooling agent is present in an amount of between 0.01 and 2 wt% based on the total weight of the content.

2. A smokeless article according to claim 1, wherein the second flavourant is menthol or comprises a flavour component providing a flavour selected from rosemary, peppermint, frankincense, spearmint, wintergreen, tea tree oil, citrus, oregano, bitter orange, grapefruit, sweet orange, green tea, violet, frankincense, anise, apple, apricot, banana, berries, guava, grape, melon, papaya, peach, pear, peas, clove, ginger, mustard, nuts, honey, coconut, passion fruit, olive, avocado, plum, rose apple, starfruit, sesame seed, tamarind, fig, cardamom, coriander seed and leaf, quince, lychee, dill, lovage, kiwi, loquat, sage, Chinese quince, chilli, nutmeg, cinnamon, lavender, basil, cocoa, vanilla, spice, star anise, fennel, and tobacco;
optionally the second flavourant is menthol or a flavour component providing a flavour selected from peppermint, spearmint, and wintergreen.

3. A smokeless article according to claim 1 or 2, wherein the cooling agent comprises one or more selected from: WS-3, WS-5, WS-23, WS-12, icilin, camphor, borneol, isoborneol, thymol, and carvone;
optionally, the cooling agent is WS-5.

4. A smokeless article according to any preceding claim, wherein the second flavourant is menthol and the cooling agent is WS-5.

5. A smokeless article according to any preceding claim, wherein the first flavourant consists of:
(i) natural eucalyptus;
(ii) synthetic eucalyptus;
(iii) artificial eucalyptus; or
(iv) natural eucalyptus and synthetic eucalyptus.

6. A smokeless article according to any preceding claim, wherein the first flavourant comprises or consists of eucalyptol, optionally wherein the first flavourant comprises 70 wt% or more of eucalyptol based on the total weight of the first flavourant..

7. A smokeless article according to any preceding claim, further comprising a third flavourant comprising one or more flavour components providing a flavour selected from rosemary, frankincense, wintergreen, tea tree oil, citrus, oregano, bitter orange, grapefruit, sweet orange, green tea, violet, frankincense, anise, apple, apricot, banana, berries, guava, grape, melon, papaya, peach, pear, peas, clove, ginger, mustard, nuts, honey, coconut, passion fruit, olive, avocado, plum, rose apple, starfruit, sesame seed, tamarind, fig, cardamom, coriander seed and leaf, quince, lychee, dill, lovage, kiwi, loquat, sage, Chinese quince, chilli, nutmeg, cinnamon, lavender, basil, cocoa, vanilla, spice, star anise, fennel, and tobacco;
wherein:
the flavour of the third flavourant is different to the flavour of the second flavourant; and
each of the first flavourant, the second flavourant, and the third flavourant is independently present in an amount of between 0.1 and 10 wt% based on the total weight of the content.

8. A smokeless article according to any preceding claim, wherein the weight ratio of the second flavourant to the first flavourant is between 0.1 and 10.

9. A smokeless article according to any preceding claim, wherein the total amount of flavourant is between 0.5 wt% and 20 wt%, optionally between 1 wt% and 10 wt%, based on the total weight of the content.

10. A smokeless article according to any one of the preceding claims, wherein the content further comprises a pentose monosaccharide in an amount of 0.1 wt% and 8 wt% based on the total weight of the content, wherein the pentose monosaccharide is one or more selected from the group consisting of arabitol, rabitol, and xylitol.

11. A smokeless article according to any preceding claim, wherein the content further comprises coloured particles, optionally wherein the particles are blue or green.

12. A method of manufacturing a smokeless article for oral consumption, the smokeless article comprising a pouch enclosing a content, wherein the method comprises the steps:
(a) mixing a nicotinic compound, at least one fibrous material, a first flavourant comprising eucalyptus flavourant, a second flavourant comprising a non-eucalyptus flavourant, and a cooling agent distinct from menthol and eucalyptol, to form a content; and
(b) adding the content to the pouch or forming the pouch around the content.

13. A method according to claim 12, wherein the first flavourant and the second flavourant are mixed together before step (a) to form a flavourant premix, and the flavourant premix is added to the mixture during step (a).

14. A method according to claim 12 or 13, wherein the second flavourant is menthol and/or the cooling agent is WS-5.

15. A kit comprising a plurality of smokeless articles according to any one of claims 1 to 11, or a plurality of smokeless articles produced according to any one of claims 12 to 14,
optionally wherein the kit further comprises a container for removably enclosing the plurality of smokeless articles.
